# FASCICULE DE BREVET EUROPEEN

(11) **EP 1 309 594 B1**
(45) Date de publication et mention de la délivrance du brevet: **15.12.2004**
(21) Numéro de dépôt: 01960871.0
(22) Date de dépôt: 06.08.2001
(51) Int. Cl.: C07D 471/06, A61K 31/4375, A61K 31/55, C07D 487/06, A61P 9/00

(54) **DERIVES DE BENZIMIDAZOLE, LEUR PREPARATION ET LEUR APPLICATION EN THERAPEUTIQUE**
BENZIMIDAZOLDERIVATE, IHRE HERSTELLUNG UND THERAPEUTISCHE VERWENDUNG
BENZIMIDAZOLE DERIVATIVES, PREPARATION AND THERAPEUTIC USE THEREOF

(30) Priorité: 08.08.2000 FR 0010419; 15.11.2000 FR 0014696
(43) Date de publication de la demande: 14.05.2003
(73) Titulaire: Sanofi-Aventis, 75013 Paris (FR)
(72) Inventeur: BARTH, Francis, F-34680 Saint-Georges d'Orques (FR); BICHON, Daniel, F-34000 Montpellier (FR); BOLKENIUS, Frank, 7640 Kehl (DE); VAN DORSSELAER, Viviane, F-67100 Strasbourg (FR)
(74) Mandataire: Thouret-Lemaitre, Elisabeth
(86) Numéro de dépôt international: PCT/FR2001/002556
(87) Numéro de publication internationale: WO 2002/012239

(56) Documents cités:
- EP-A- 0 646 583
- EP-A- 0 732 334
- WO-A-00/32579

## Description

La présente invention a pour objet des dérivés de benzimidazole, leur préparation La présente invention a pour objet des dérivés de benzimidazole, leur préparation et leur application en thérapeutique.

La présente invention a pour objet les composés répondant à la formule (I) : 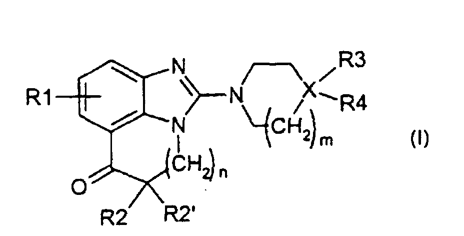 dans laquelle:
**R1** représente un atome d'hydrogène, un groupe (C1-C4)alkyle, un atome d'halogène, un groupe nitro ou un groupe (C1-C4)alcoxy,
**R2** et **R2'** représentent indépendamment l'un de l'autre un atome d'hydrogène ou un groupe (C1-C4)alkyle,
**X** représente un atome d'azote ou un atome de carbone,
**n** est égal à 1 ou 2,
**m** est égal à 1 ou 2,
**et dans le cas où X représente un atome d'azote :**
**R3** représente un atome d'hydrogène ou un groupe (C1-C4)alkyle pour donner des composés de formule (I) comportant un ammonium quaternaire ou encore n'existe pas pour donner des composés de formule (I) comportant une amine secondaire ou tertiaire,
**R4** représente
   un atome d'hydrogène,
   un groupe (C1-C6)alkyle,
   un groupe (C3-C7)cycloalkyle,
   un groupe (C3-C7)hétérocycloalkyle éventuellement substitué par un groupe (C1-C4)alkyle ou un groupe -COOR, où R représente un groupe (C1-C6)alkyle,
   un groupe -(CH₂)ₚ-hétéroaryle, où p peut varier de 0 à 4 et où le groupe hétéroaryle est choisi parmi les groupes pyridyle, aminopyridyle, pyrimidinyle, pyrazinyle, pyridazinyle, imidazolyle et thiényle, ledit groupe hétéroaryle étant éventuellement pyridazinyle, imidazolyle et thiényle, ledit groupe hétéroaryle étant éventuellement substitué par un groupe (C1-C4)alkyle,
   un groupe hétéroarylecarbonyle, le groupe hétéroaryle étant choisi parmi les groupes furyle, pyridyle, pyrimidyle, pyrazinyle, pyridazinyle et imidazolyle,
   un groupe phénylcarbonyle, le groupe phényle étant éventuellement substitué par un atome d'halogène,
   un groupe (C1-C6)alkylcarbonyle,
   un groupe -(CH₂)ₚCOOR où p peut varier de 0 à 4 et où **R** représente un groupe (C1-C6)alkyle,
   un groupe phénylsulfonyle éventuellement substitué sur le noyau phényle par un atome d'halogène, un groupe trifluorométhyle, un groupe (C1-C4)alkyle, un groupe nitro ou un groupe (C1-C4)alcoxy, ou bien
   un groupe -(CH₂)ₚ-phényle, où p peut varier de 0 à 4 et où le groupe phényle est éventuellement substitué, en position *ortho* et/ou *méta* et/ou *para,* par un à trois groupes choisis indépendamment l'un de l'autre parmi : un groupe (C1-C4)alkyle, un groupe nitro, un groupe amino, un groupe hydroxy, un atome d'halogène, un groupe trifluorométhyle, un groupe (C1-C4)alcoxy, un groupe (C1-C4)alcoxy-phényle, un groupe (C1-C4)alkylamino, un groupe (C1-C4)dialkylamino, un groupe -NHCHO ou un groupe -NHCOR', où R' représente un groupe (C1-C4)alcoxy ou un groupe (C1-C4)alkyle, ce groupe (C1-C4)alkyle pouvant être substitué par un groupe diméthylamino,
**et dans le cas où X représente un atome de carbone :**
**R3** représente un atome d'hydrogène, un groupe -NR5R6, un groupe -N(R5)₃⁺, un groupe -NHCOR7, un groupe -CONHR5, un groupe -COR7, un groupe -NHCONH₂, un groupe -OH ou un groupe -CH₂OH,
**R4** représente
   un atome d'hydrogène,
   un groupe -(CH₂)ₚ-phényle, où p peut varier de 0 à 4 et où le groupe phényle est éventuellement substitué par un à trois groupes choisis indépendamment l'un de l'autre parmi : un groupe (C1-C4)alkyle, un groupe nitro, un groupe amino, un atome d'halogène, un groupe trifluorométhyle ou un groupe (C1-C4)alcoxy,
   un groupe -(CH₂)ₚ-hétéroaryle, où p peut varier de 0 à 4 et où le groupe hétéroaryle est choisi parmi un groupe pyridyle, un groupe aminopyridyle, un groupe pyrimidinyle, un groupe pyrazinyle ou un groupe pyridazinyle, ou bien
   un groupe -(CH₂)ₜNR7R8, où t est égal à 0 ou 1,
   sous réserve que lorsque R4 représente un groupe -NR7R8, R3 est différent des groupes -NR5R6, -NHCOR7, -NHCONH₂, et -OH,
**R5** et **R6** représentent indépendamment l'un de l'autre un atome d'hydrogène ou un groupe (C1-C4)alkyle,
**R7** et **R8** représentent indépendamment l'un de l'autre un groupe (C1-C4)alkyle ou (C1-C4)alcoxy, ou forment ensemble un cycle saturé, comportant de 5 à 7 chaînons et comprenant éventuellement un atome d'azote supplémentaire, pour former par exemple un groupe pipéridin-1-yle, un groupe pyrrolidin-1-yle ou un groupe pipérazin-1-yle, ce cycle pouvant être substitué, sur un atome de carbone ou bien un atome d'azote, y compris sur l'atome d'azote sur lequel sont liés les groupes R7 et R8 pour former un ammonium quaternaire, par un groupe (C1-C4)alkyle ou un groupe -COOR", où R" représente un groupe phényle ou (C1-C4)alkylphényle.

Les deux composés de formule (I) pour lesquels R1 = R2 = R2' = R3 = H; X = C, n = m = 1 et **R4** représente soit un groupe imidazol-4-yle, soit un groupe 5-méthyl-imidazol-4-yle sont divulgués dans la demande de brevet EP 646 583, comme composés n°16 et 17 du tableau, en tant que ligands des récepteurs sérotoninergiques de types 5-HT₃ et 5-HT₄.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de l'invention peuvent exister à l'état de bases ou de sels d'addition à des acides pharmaceutiquement acceptables. De tels sels d'addition font également partie de l'invention.

Dans le cadre de la présente invention, on entend par :
- un groupe (Cq-Cr)alkyle, un groupe aliphatique saturé linéaire ou ramifié comportant de q à r atomes de carbone, q et r étant des nombres entiers ; on peut notamment citer les groupes méthyle, éthyle, propyle, isopropyle, n-propyle, butyle, isobutyle, tertbutyle, n-butyle, pentyle, etc ;
- un atome d'halogène, un fluor, un chlore, un brome ou un iode ;
- un groupe (C3-C7)cycloalkyle, un groupe alkyle cyclique comportant de 3 à 7 atomes de carbone ; on peut notamment citer les groupes cyclopropyle, cyclobutyle, cyclopentyle, cyclohexyle ou cycloheptyle ;
- un groupe (C3-C7)hétérocycloalkyle, un groupe alkyle cyclique comportant de 3 à 7 atomes de carbone ainsi qu'un ou plusieurs hétéroatomes, par exemple un atome d'azote ; on peut notamment citer les groupes pipéridyles;
- un groupe (C1-C4)alcoxy-phényle, un groupe de formule -O-(CH₂)ₓ-phényle, où x peut varier de 1 à 4.

Parmi les composé de formule (I) objets de la présente invention on peut citer les composés préférés pour lesquels :
**R1** représente un atome d'hydrogène, un groupe (C1-C4)alkyle ou un groupe (C1-C4)alcoxy,
**R2** représente un atome d'hydrogène ou un groupe (C1-C4)alkyle,
**R2'** représente un atome d'hydrogène,
**X** représente un atome d'azote,
**n** est égal à 1 ou 2,
**m** est égal à 1 ou 2,
**R3** représente un atome d'hydrogène pour donner des composés de formule (I) comportant un ammonium quaternaire ou encore n'existe pas pour donner des composés de formule (I) comportant une amine secondaire ou tertiaire,
**R4** représente
   un atome d'hydrogène,
   un groupe (C1-C6)alkyle,
   un groupe (C3-C7)cycloalkyle,
   un groupe pyridyle, pyrimidinyle ou pyrazinyle, éventuellement substitué par un groupe (C1-C4)alkyle,
   un groupe hétéroarylecarbonyle, le groupe hétéroaryle étant choisi parmi un groupe furyle ou un groupe pyridyle,
   un groupe phénylcarbonyle, le groupe phényle pouvant éventuellement être substitué par un atome d'halogène,
   un groupe (C1-C6)alkylcarbonyle,
   un groupe -(CH₂)ₚCOOR où p peut varier de 0 à 4 et où **R** représente un groupe (C1-C6)alkyle,
   un groupe phénylsulfonyle,
   un groupe phényle substitué par un à trois groupes choisis indépendamment l'un de l'autre parmi : un groupe (C1-C4)alkyle, un groupe nitro, un groupe amino, un groupe hydroxy, un atome d'halogène, un groupe trifluorométhyle, un groupe (C1-C4)alcoxy, un groupe (C1-C4)alcoxy-phényle, un groupe (C1-C4)dialkylamino, un groupe -NHCHO ou un groupe -NHCOR', où **R'** représente un groupe (C1-C4)alcoxy ou un groupe (C1-C4)alkyle, ce groupe (C1-C4)alkyle pouvant être substitué par un groupe diméthylamino,
   un groupe -(CH₂)ₚ-phényle, où p peut varier de 0 à 4,
   un groupe -(CH₂)ₚ-pyridyle, où p peut varier de 0 à 4,
   un groupe -(CH₂)ₚ-thiényle, où p peut varier de 0 à 4,
   un groupe (C3-C7)hétérocycloalkyle éventuellement substitué par un groupe (C1-C4)alkyle ou un groupe -COOR, où **R** représente un groupe (C1-C6)alkyle,
   ou bien encore les composés préférés pour lesquels :
**R1** représente un atome d'hydrogène,
**R2** et **R2'** représentent indépendamment l'un de l'autre un atome d'hydrogène ou un groupe (C1-C4)alkyle,
**X** représente un atome de carbone,
**n** est égal à 1 ou 2,
**m** est égal à 1,
**R3** représente un atome d'hydrogène, un groupe -NR5R6, un groupe -N(R5)₃⁺, un groupe -NHCOR7, un groupe -CONHR5, un groupe -NHCONH₂, un groupe -OH ou un groupe -CH₂OH,
**R4** représente
   un atome d'hydrogène,
   un groupe benzyle (c'est-à-dire un groupe -(CH₂)ₚ-phényle dans lequel p est égal à 1),
   un groupe phényle éventuellement substitué par un à trois groupes choisis indépendamment l'un de l'autre parmi : un groupe (C1-C4)alkyle, un groupe nitro, un groupe amino, un atome d'halogène, un groupe trifluorométhyle ou un groupe (C1-C4)alcoxy,
   un groupe pyridyle,
   un groupe -NR7R8,
   sous réserve que lorsque R4 représente un groupe -NR7R8, R3 est différent des groupes -NR5R6, -NHCOR7, -NHCONH₂, et -OH,
**R5** et **R6** représentent indépendamment l'un de l'autre un atome d'hydrogène ou un groupe (C1-C4)alkyle,
**R7** et **R8** représentent indépendamment l'un de l'autre un groupe (C1-C4)alkyle ou (C1-C4)alcoxy, ou forment ensemble un cycle saturé, comportant de 5 à 7 chaînons et comprenant éventuellement un atome d'azote supplémentaire, ce cycle pouvant être substitué, sur un atome de carbone ou bien un atome d'azote, y compris sur l'atome d'azote sur lequel sont liés les groupes R7 et R8 pour former un ammonium quaternaire, par un groupe (C1-C4)alkyle ou un groupe -COOR", où **R"** représente un groupe phényle ou (C1-C4)alkylphényle.

Parmi ces derniers composés préférés, on préfère tout particulièrement les composés de formule (I) pour lesquels :
**R1** représente un atome d'hydrogène, un groupe méthyle ou un groupe méthoxy,
**R2** représente un atome d'hydrogène ou un groupe méthyle,
**R2'** représente un atome d'hydrogène,
**X** représente un atome d'azote,
**n** est égal à 1 ou 2,
**m** est égal à 1 ou 2,
**R**_{**3**} représente un atome d'hydrogène pour donner des composés de formule (I) comportant un ammonium quaternaire ou encore n'existe pas pour donner des composés de formule (I) comportant une amine secondaire ou tertiaire,
**R4** représente
   un atome d'hydrogène,
   un groupe (C1-C4)alkyle,
   un groupe (C6-C7)cycloalkyle,
   un groupe pyridyle, pyrimidinyle ou pyrazinyle, éventuellement substitué par un groupe (C1-C4)alkyle,
   un groupe hétéroarylecarbonyle, le groupe hétéroaryle étant choisi parmi un groupe furyle ou un groupe pyridyle,
   un groupe phénylcarbonyle, le groupe phényle pouvant éventuellement être substitué par un atome d'halogène,
   un groupe (C3-C5)alkylcarbonyle,
   un groupe -(CH₂)ₚCOOR où p est égal à 0 ou 1 et où **R** représente un groupe (C1-C4)alkyle,
   un groupe phénylsulfonyle,
   un groupe phényle substitué par un à trois groupes choisis indépendamment l'un de l'autre parmi : un groupe méthyle, un groupe nitro, un groupe amino, un groupe hydroxy, un atome d'halogène, un groupe trifluorométhyle, un groupe méthoxy, un groupe (C1-C4)alcoxy-phényle, un groupe diméthylamino, un groupe -NHCHO ou un groupe -NHCOR', où **R'** représente un groupe (C1-C4)alcoxy ou un groupe (C1-C4)alkyle, ce groupe (C1-C4)alkyle pouvant être substitué par un groupe diméthylamino,
   un groupe -(CH₂)ₚ-phényle, où p est égal à 1, 2, 3 ou 4,
   un groupe -(CH₂)ₚ-pyridyle, où p peut varier de 1 à 3,
   un groupe -(CH₂)ₚ-thiényle, où p est égal à 2,
   un groupe (C6-C7)hétérocycloalkyle éventuellement substitué par un groupe méthyle ou un groupe -COOR, où **R** représente un groupe (C1-C4)alkyle,
   ou bien encore on préfère tout particulièrement les composés de formule (I) pour lesquels :
**R1** représente un atome d'hydrogène,
**R2** et **R2'** représentent indépendamment l'un de l'autre un atome d'hydrogène ou un groupe méthyle,
**X** représente un atome de carbone,
**n** est égal à 1 ou 2,
**m** est égal à 1,
**R3** représente un atome d'hydrogène, un groupe -NR5R6, un groupe -N(CH₃)₃⁺, un groupe -NHCOR7, un groupe -CONHR5, un groupe -NHCONH₂, un groupe -OH ou un groupe -CH₂OH,
**R4** représente
   un atome d'hydrogène,
   un groupe benzyle,
   un groupe phényle éventuellement substitué par un à trois groupes choisis indépendamment l'un de l'autre parmi un atome d'halogène ou un groupe trifluorométhyle,
   un groupe pyridyle,
   un groupe -NR7R8,
   sous réserve que lorsque R4 représente un groupe -NR7R8, R3 est différent des groupes -NR5R6, -NHCOR7, -NHCONH₂, et -OH,
**R5** et **R6** représentent indépendamment l'un de l'autre un atome d'hydrogène ou un groupe (C1-C4)alkyle,
**R7** et **R8** représentent indépendamment l'un de l'autre un groupe (C1-C4)alkyle ou forment ensemble un cycle saturé, comportant de 5 à 7 chaînons et comprenant éventuellement un atome d'azote supplémentaire, ce cycle pouvant être substitué, sur un atome de carbone ou bien un atome d'azote, y compris sur l'atome d'azote sur lequel sont liés les groupes **R7** et **R8** pour former un ammonium quaternaire, par un groupe méthyle ou un groupe -COOR", où **R"** représente un groupe (C1-C4)alkylphényle.

Dans ce qui suit, on entend par « groupe partant » un groupe pouvant être facilement clivé d'une molécule par rupture d'une liaisons hétérolytique, avec départ d'une paire électronique. Ce groupe peut ainsi être remplacé facilement par un autre groupe lors d'une réaction de substitution par exemple. De tels groupes partants sont, par exemple, les halogènes, ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3^{rd} Edition, Wiley Interscience, p. 310-316.

Pour préparer les composés de formule (I) conformes à l'invention, on procède selon le schéma de synthèse 1 ci-dessous. Selon ce procédé, on fait réagir un dérivé de formule (II), dans laquelle R1, R2, R2' et n sont tels que définis précédemment et A représente un groupe partant, de préférence un halogène, en présence d'une amine de formule (III), dans laquelle X, R3, R4 et m sont tels que définis précédemment, dans un solvant qui peut être un alcool, tel que l'alcool isoamylique, un éther tel que le tétrahydrofurane ou le TGME (triéthylèneglycol monométhyléther) ou bien un hydrocarbure tel que le toluène, à une température comprise entre la température ambiante et la température d'ébullition du solvant, pour obtenir le composé de formule (I). La réaction peut s'effectuer en présence d'une base telle que la 2,6-diméthyllutidine ou le tertiobutylate de sodium, en présence d'halogénures de métal alcalin tel le fluorure de potassium ou en présence de catalyseurs à base de palladium ou de nickel, comme décrit par exemple dans la demande de brevet EP 646 583 ou dans *J. Med. Chem.* (1986) **29** 1178-1183, *Tetrahedron Letters* (1997) **32** 5607-5610, *Tetrahedron Letters* (1999) **55** 12829-12842 et *Tetrahedron Letters* (1999) **40** 6875-6879.

Lorsque le composé de formule (I) comporte une fonction amine primaire ou secondaire libre, il peut également être obtenu par réaction d'un dérivé de formule (II) avec une amine de formule (III) dans laquelle ladite fonction amine est protégée par un groupe classique de protection d'amine tel qu'un tertiobutylcarbamate (BOC). Le composé de formule (I) à fonction amine protégée ainsi obtenu est ensuite traité selon une des méthodes connues pour obtenir le composé (I) à fonction amine libre désiré. Des

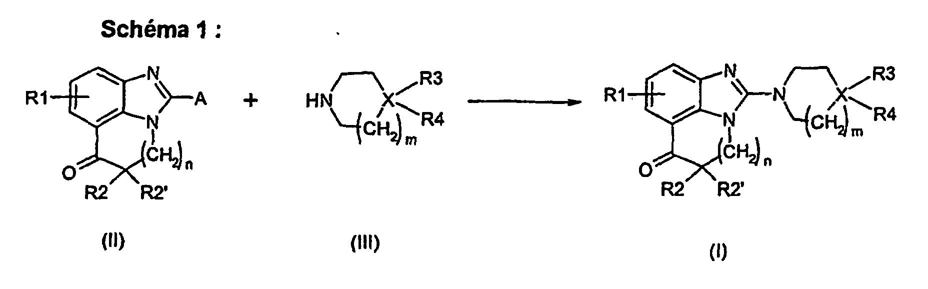

Les composés de formule (II) peuvent être préparés d'après le schéma 2 ci-dessous, selon des conditions opératoires connues de l'homme du métier, en particulier par réaction d'un composé de formule (IV), dans laquelle R1, R2, n et R2' sont tels que définis précédemment, avec un agent d'halogénation tel que le chlorure de phosphoryle.

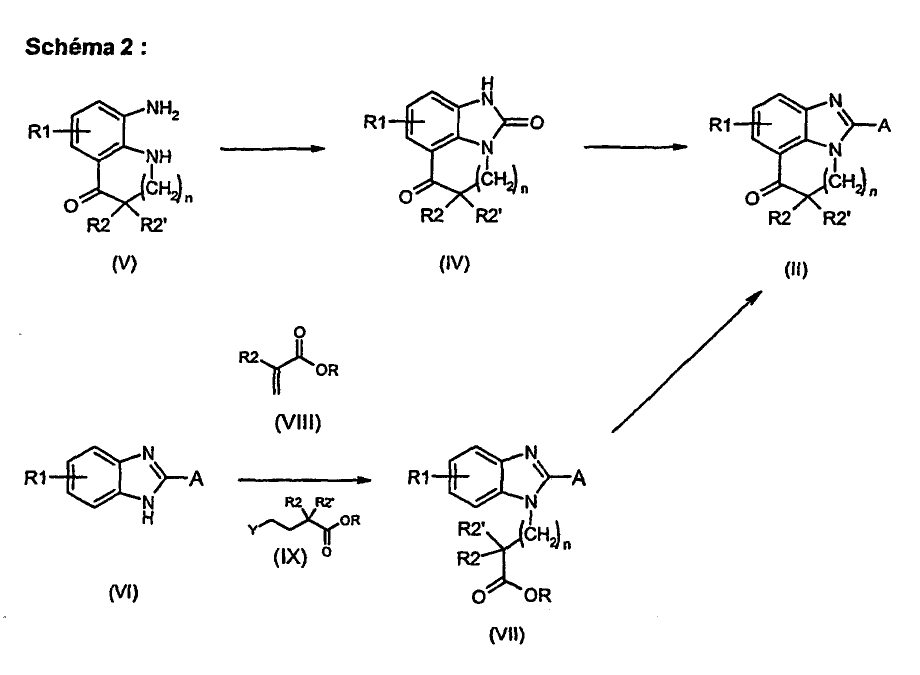

Les composés de formule (IV) peuvent être préparés selon un procédé décrit dans le schéma de synthèse 2 ci-dessus. Selon une variante de ce procédé, une diamine de formule (V), dans laquelle R1, R2, R2' et n sont tels que définis précédemment, est condensée avec un dérivé du phosgène tel que le carbonyle diimidazole (CDI). Selon une autre variante, un dérivé de formule (VI), dans laquelle R1 est tel que défini précédemment, est alkylé par un agent alkylant de formule (VIII, R= (C1-C4)alkyle) dans laquelle R2 est tel que défini précédemment pour obtenir les produits de formule (VII) où n=1 et R2'=H, ou par un agent de formule (IX, R= (C1-C4)alkyle et Y est un groupe partant), dans laquelle R2 et R2' sont tels que définis précédemment, pour obtenir les produits de formule (VII) où n=2. Les composés (VII) ainsi obtenus sont ensuite transformés en acides carboxyliques (VII, R=H) ou en dérivés d'acides tel que des chlorures d'acides (VII, R=CI) puis cyclisés selon des conditions opératoires connues de l'homme de métier pour obtenir directement les intermédiaires de formule (II). Une approche similaire à cette deuxième variante est notamment décrite dans la demande de brevet JP 55111406 ou dans *Tetrahedron Letters* (1995) **36** 1387-1390.

Alternativement, les composés de formule, (II) dans lesquels R2 et R2' ne représentent pas des atomes d'hydrogène peuvent être préparés à partir des composés (II) correspondants dans lesquels R2' représente un atome d'hydrogène par alkylation avec un réactif de type R2'Z dans lequel Z représente un groupe partant, de préférence l'iode. Cette réaction peut s'effectuer dans un solvant de type diméthylformamide, éther ou tétrahydrofurane en présence d'une base, selon les méthodes connues de l'homme de métier.

Les composés de formules (III), (V), (VI), (VIII) et (IX) sont disponibles dans le commerce ou peuvent être préparés selon des conditions opératoires connues de l'homme du métier.

La présente invention a aussi pour objet les intermédiaires de synthèse de formule (II) qui sont nouveaux.

Les exemples suivants illustrent la présente invention. Les numéros des composés exemplifiés renvoient à ceux du tableau donné plus loin, qui illustre les structures chimiques de quelques composés selon l'invention.

### Exemple 1 : Préparation d'intermédiaires de formule (II).

### 1.1 Préparation de 2-chloro-4,5-dihydro-imidazo[4,5,1-ij]quinoléin-6-one (A = Cl, R1 = H, R2 = R2' = H, n = 1)

Ce composé est obtenu à partir du composé 4*H*-imidazo[4,5, -ij]-quinoléin-2,6-(1*H*,5*H*)dione (IV), décrit dans le brevet Japonais : JP 55111406. Dans un tricol de 250 ml, équipé d'un reflux, on fait réagir au reflux 10 g de composé (IV) avec 38,6 ml d'oxychlorure de phosphore et 6,3 g de chlorure d'ammonium, pendant 1,5 heure. Le mélange réactionnel est ensuite refroidi, et versé sur de la glace à laquelle on ajoute une solution d'ammoniaque à 20%, sous forte agitation, jusqu'à obtention d'un pH=9. On extrait le mélange avec 2 fois 250 ml d'acétate d'éthyle, on sèche sur sulfate de magnésium, filtre et évapore: On obtient 9,81 g d'un solide blanc qu'on utilise tel quel dans l'étape suivante. RMN ¹H (200 MHz, δ ppm) DMSO D6 : 7,8 (d, 1H), 7,5 (d, 1H), 7,3 (t, 1H), 4,5 (t, 2H), 3,0 ( t, 2H).

### 1.2 Préparation de 1-chloro-8,9-dihydro-7H-2,9a-diaza-benzo[cd]azulen-6-one (A = Cl, R1 = H, R2 = R2' = H, n = 2)

### 1.2.1 : Ester éthylique de l'acide 3-(2-chloro-benzimidazole-1-yl) butyrique

Dans un tricol de 1L avec agitation magnétique, ampoule d'addition et réfrigérant, on introduit sous atmosphère d'azote l'hydrure de sodium à 60% en dispersion dans l'huile (2,88 g, 72 mmoles), le lave 2 fois au pentane, rajoute un peu de diméthylformamide puis une solution de 4-bromobutyrate d'éthyle (14,78 g, 72 mmoles) dans de la diméthylformamide anhydre (200 ml). Après agitation pendant 1 heure à température ambiante, on additionne au mélange le 2-chloro-1H-benzimidazole (10,0 g, 65,5 mmoles) en solution dans de la diméthylformamide anhydre (200 ml). On chauffe le mélange réactionnel à 65°C pendant 8 heures puis on le garder à température ambiante durant la nuit. Après évaporation de la diméthylformamide, on reprend le résidu à l'acétate d'éthyle, lave la phase organique avec du chlorure de sodium saturé, la sèche sur sulfate de magnésium, filtre et concentre. Une flash chromatographie sur gel de silice (750 g) du brut (22 g) avec un gradient d'élution de 10 à 30 % d'acétate d'éthyle dans de l'éther de pétrole permet d'obtenir le composé du titre sous forme d'une huile jaune (16,58 g, 95%). RMN ¹H (300 MHz, δ ppm) CDCl₃: 1,25 (t, 3H), 2,15 (quint., 2H), 2,40 (t, 2H), 4,15 (quad., 2H), 4,25, (t, 2H), 7,20- 7,40 (m, 2H), 7,70 (dd, 1H).

### 1.2.2 : Acide 3-(2-chloro-benzimidazole-1-yl)butyrique, sel de lithium

Dans un ballon monocol de 1L avec agitation magnétique, on introduit l'ester éthylique (16,58 g, 62,2 mmoles) en solution dans du tétrahydrofurane (180 ml) puis ajoute une solution aqueuse d'hydroxyde de lithium (1,49 g, 24 mmoles, 100 ml d'eau distillée). On laisser réagir pendant la nuit à température ambiante, évapore le tétrahydrofurane et l'eau puis reprend le résidu dans de l'éther éthylique (2L) et agite pendant 2 heures. On filtre le précipité blanc obtenu, le lave à l'éther éthylique puis le sèche très soigneusement sous vide de pompe à palettes sur pentoxyde de phosphore pour obtenir le composé attendu sous forme de cristaux blancs (14 g, 92%). Le composé est utilisé tel quel pour l'étape suivante. RMN ¹H (300 MHz, δ ppm) DMSO D6 + ε D₂O: 1,85 (m, 2H), 1,95 (m, 2H), 4,20 (t, 2H), 7,25 (m, 2H), 7,55 (d, 1 H), 7,60 (d, 1H). LC-MS: MH⁺ = 239 (acide).

### 1.2.3 : 1-chloro-8,9-dihydro-7H-2,9a-diaza-benzo[cd]azulen-6-one

Dans un tricol de 2L sous atmosphère d'argon, avec agitation mécanique, réfrigérant et ampoule d'addition on introduit le sel de lithium (11,95 g, 49,7 mmoles) puis ajoute du 1,2-dichloroéthane fraîchement distillé sur pentoxyde de phosphore (1L). On ajoute rapidement sous agitation le chlorure d'oxalyle (8,55 ml, 102 mmoles) et chauffe le mélange réactionnel pendant 15 min vers 40°C. On ajoute au chlorure d'acide intermédiaire ainsi obtenu, le chlorure d'aluminium (19,54 g, 154,5 mmoles) et chauffe le mélange au reflux pendant 3 heures. On laisse refroidir puis verse sur un mélange glace/sel et extrait au 1,2-dichloroéthane, lave la phase organique avec une solution saturée en chlorure de sodium, sèche sur sulfate de magnésium, filtre puis évapore le solvant. Une flash chromatographie du brut (10,6 g) sur gel de silice (800 g) avec élution par 20% d'acétate d'éthyle dans du dichlorométhane permet l'obtention du produit attendu sous forme de cristaux blancs (7,06 g, 65 %). RMN ¹H (300 MHz, δ ppm) CDCl₃: 2,40 (quint., 2H), 3,15 (t, 2H), 4,40, (t, 2H), 7,35 (t, 1H), 7,95 (d, 1H), 8,05 (d, 1H). LC-MS: MH⁺ = 221.

### 1.3 Préparation de 2-chloro-5-méthyl-4,5-dihydro-imidazo[4,5,1-ij] quinoléin-6-one (A = Cl, R1 = H, R2 = CH₃, R2' = H, n = 1)

### 1.3.1 : Ester méthylique de l'acide 1-méthyl-3-(2-chloro-benzimidazole-1-yl) propionique.

Dans un tricol de 1L sous atmosphère d'azote avec agitation magnétique, ampoule d'addition et réfrigérant, on introduit le 2-chloro-1*H*-benzimidazole (15,25 g, 100 mmoles) en solution dans du chloroforme (100 ml). On ajoute ensuite le Triton B (47 ml, 110 mmoles) et le méthacrylate de méthyle (107 ml, 1 mole). On maintient la réaction au reflux pendant 2 heures, laisse refroidir puis évapore le chloroforme. On reprend le résidu par de l'acétate d'éthyle, lave la phase organique 3 fois à l'eau et 1 fois avec une solution saturée de chlorure de sodium. Une flash chromatographie du brut (16,0 g) sur du gel de silice (1 kg) avec un gradient d'élution 30 à 70 % d'acétate d'éthyle dans de l'éther de pétrole permet l'obtention du produit attendu sous forme de cristaux blancs (10,1 g, 40%). RMN ¹H (300 MHz, δ ppm) DMSO D6: 1,15, (d, 3H), 3,05 (m, 1H), 3,50 (s, 3H), 4,25 (dd, 1H), 4,50 (dd, 1H), 7,25 (m, 2H), 7,60 (m, 2H).

### 1.3.2 : Acide 1-méthyl-3-(2-chloro-benzimidazole-1-yl) propionique, sel de lithium.

On obtient ce composé selon la méthode décrite dans la préparation au point 1.2 en traitant l'ester méthylique de l'acide 1-méthyl-3-(2-chloro-benzimidazole-1-yl) propionique par de l'hydroxyde de lithium. RMN ¹H (300 MHz, δ ppm) DMSO D6 +_{ε} D₂O: 1,35 (d, 3H), 2,60 (m, 1H), 4,10 (dd, 1H), 4,40 (dd, 1H), 7,25 (m, 2H), 7,55 (d, 1H), 7,60 (d, 1H). LC-MS: MH⁺ = 239 (acide).

### 1.3.3 : 2-chloro-5-méthyl-4,5-dihydro-imidazo[4,5,1-ij]quinoléin-6-one.

On obtient ce composé à partir du sel de lithium selon la méthode décrite au point 1.2, en le traitant successivement par du chlorure d'oxalyle qui permet de préparer le chlorure d'acide intermédiaire, et par du chlorure d'aluminium. RMN ¹H (300 MHz, δ ppm) DMSO D6: 1,40 (d, 3H), 3,20 (m, 1H), 4,10 (dd, 1H), 4,65 (dd, 1H), 7,45 (t, 1H), 7,70 (d, 1H), 7,85 (d, 1H). LC-MS: MH⁺ = 221.

### 1.4. Préparation de 2-chloro-4,5-dihydro-9-méthyl-imidazo[4,5,1-ij]quinoléin-6-one (A = Cl, R1 = 9-CH₃, R2 = R2' = H, n = 1)

### 1.4.1. 2-hydroxy-4-méthyl-benzimidazole.

Dans un bicol de 250 ml sous atmosphère d'argon avec agitation magnétique, on introduit successivement le 2,3-diaminotoluène (5g, 41 mmoles), le 1,1-carbonyl-diimidazole (7,3 g, 45 mmoles) et 50 mL de DMF anhydre. Après chauffage du mélange à 90-95°C pendant 4h, le solvant est distillé sous vide et le résidu obtenu est repris par de l'eau (250 ml) et extrait à l'acétate d'éthyle (3x250 ml). L'insoluble formé lors de l'extraction est récupéré (4,8 g) et les phases organiques réunies sont relavées avec une solution saturée en NaCl, séchées sur MgSO₄, filtrées et concentrées permettant de récupérer 1,2 g de produit désiré supplémentaire (6 g, quantitatif). RMN ¹H (300 MHz, δ ppm) DMSO D6: 2,27 (s, 3H), 6,72 (m, 2H), 6,82 (m, 1H).

### 1.4.2. 2-chloro-4-méthyl-benzimidazole.

Dans un bicol de 250 ml sous atmosphère d'argon avec agitation magnétique, on introduit le 2-hydroxy-4-méthyl-benzimidazole (5,92 g, 40 mmoles) et 40 ml de chlorure de phosphoryle. Le mélange est chauffé sous reflux pendant 20 heures et le chlorure de phosphoryle est évaporé sous vide. On reprend le solide obtenu dans de l'eau (250 ml), on neutralise jusqu'à pH=8 avec de l'ammoniaque à 28% et on extrait la phase aqueuse avec de l'acétate d'éthyle (3x250 ml). Après traitement usuel on obtient le produit du titre avec un rendement de 93% (6,23 g). RMN ¹H (300 MHz, δ ppm) DMSO D6: 2,47 (s, 3H), 7,01 (d, 1H), 7,11 (t, 1H), 7,32 (d, 1H).

### 1.4.3. Ester méthylique de l'acide 1-méthyl-3-(2-chloro-4-méthy)-benzimidazole-1-yl)propionique.

Le composé du titre est préparé selon le mode opératoire décrit dans l'exemple 1.3.1. (7,85 g, 94%). RMN ¹H (300 MHz, δ ppm) DMSO D6: 2,48 (s, 3H), 2,86 (t, 2H), 3,56 (s, 3H), 4,49 (t, 2H), 7,06 (d, 1H), 7,19 (t, 1H), 7,44 (d, 1H).

### 1.4.4. Acide 1-méthyl-3-(2-chloro-4-méthyl-benzimidazole-1-yl)propionique, sel de lithium.

Le composé du titre est préparé selon le mode opératoire décrit dans l'exemple 1.3.2. (6,94 g, 94%). RMN ¹H (300 MHz, δ ppm) DMSO D6: 2,3 (dd, 2H), 2,48 (s, 3H), 4,32 (dd, 2H), 7,01 (d, 1H), 7,15 (t, 1H), 7,41 (d, 1H) (acide).

### 1.4.5. 2-chloro-4,5-dihydro-9-méthyl-imidazo[4,5,1-ij]quinoléin-6-one.

Le composé du titre est préparé selon le mode opératoire décrit dans l'exemple 1.3.3. (4,72 g, 76%). RMN ¹H (300 MHz, δ ppm) DMSO D6: 2,58 (s, 3H), 3,05 (t, 2H), 4,53 (t, 2H), 7,19, d, 1H), 7,52 (d, 1H).

### Exemple 2 : 1-(6-oxo-5,6-dihydro-4H-imidazo[4,5,1-ij]quinoléin-2-yl)-4-pipéridinopipéridine (R1 = R2 = R2' = R3 = H, R4= pipéridin-1-yl, X = C, n = m = 1) (composé n° 45)

On fait réagir 400 mg de dérivé chloré de formule (II) décrit au point 1.1, 5 ml de diméthylformamide, 70 mg de fluorure de potassium, 0,2 ml de 2,6 lutidine et 500 mg de 4-pipéridinopipéridine, pendant 2 heures à 140°C. Après évaporation du solvant, le résidu est chromatographié sur gel de silice (éluant: dichlorométhane + 8% méthanol). On obtient 375 mg de produit sous la forme d'un solide blanchâtre. RMN ¹H (200 MHz, δ ppm) DMSO D6: 1,2-2,2 (m, 12H) 3,0 (m, 3H), 3,2-3,4 (m, 4H), 4,2 (d, 2H), 4,65 (t, 2H), 7,4 (t, 1H), 7,6, (d, 1H), 7,8 (d, 1H).

### Exemple 3 : Chlorure de 1-méthyl-1-[1-(6-oxo-5,6-dihydro-4H-imidazo[4,5,1-ij]quinoléin-2-yl)-4-pipéridinyl]-pipéridinium (R1 = R2 = R2' = R3 = H, R4 = N-méthyl-pipéridin-1-yl, X = C, n = 1, m = 1) (composé n° 48)

### 3.1 : 4-(N-pipéridino) Boc pipéridine

Dans un ballon tricol sous azote, on mélange 3,98 g de 4-oxo-N Boc pipéridine, 1,7 g de pipéridine, 7,1 g de Ti (IV) isopropoxide et on laisse sous agitation pendant une heure à température ambiante. On ajoute ensuite 20 ml d'éthanol absolu et 850 mg de NaBH₃CN. On laisse réagir 17 heures à température ambiante, ajoute 5 ml d'eau, laisse agiter 5 minutes et filtre sur papier Whatman. La solution rose est évaporée, reprise dans 100 ml d'acétate d'éthyle, séchée sur sulfate de magnésium, et évaporée. Le résidu obtenu est chromatographié sur 100 g de Silice et élué par l'acétone. On obtient 2,2 g d'huile, qui cristallise, dont le spectre RMN correspond à la structure attendue et est utilisée telle quelle pour la suite.

### 3.2 : Chlorure de N-Boc-4-(N-méthyl-pipéridinium) pipéridine

Le composé obtenu ci-dessus (2,2 g) est mis sous agitation avec 5 ml de CH₃l pendant 17 heures à l'abri de la lumière, puis évaporé. On obtient 3,10 g de cristaux blancs, utilisés tels quels dans l'étape suivante.

### 3.3 : Chlorure de 4-(N-méthyl-pipéridinium)pipéridine

Les cristaux obtenus ci-dessus sont mis en suspension dans 20 ml de dichloromethane dans lequel on ajoute 5 ml d'une solution saturée d'acide chlorhydrique dans l'éther. Le précipité gommeux obtenu est redissout dans 20 ml de méthanol, évaporé à sec et repris dans 5 ml de méthanol. On refroidit à 0°C et ajoute 1,35 ml de méthanolate de sodium (à 30 % dans le méthanol), laisse agiter 15 minutes, filtre et évapore à la pompe à vide. L'amine obtenue est utilisée telle quelle pour le couplage suivant.

### 3.4: Chlorure de 1-méthyl-1-[1-(6-oxo-5,6-dihydro-4H-imidazo[4,5,1-ij]quinoléin-2-yl)-4-pipéridinyl]-pipéridinium

Dans un tricol, sous argon, on ajoute 400 mg de 2-chloro-4,5-dihydro-6*H*-imidazo[4,5,1-ij]quinoléin-6-one dans 5 ml de diméthylformamide, 70 mg de fluorure de potassium, 0,2 ml de 2,6 lutidine, 1,6 g de chlorure de 4-(*N*-méthyl-piperidinium) pipéridine, et on laisse sous agitation 5 heures à 140°C. On évapore à la pompe à vide et chromatographie le résidu obtenu avec 70 g de Silice H (Merck) (éluant dichlorométhane avec 10% de méthanol et 0,5% de triéthylamine). On obtient 50 mg de produit pur dont le spectre RMN correspond à la structure recherchée.

### Exemple 4: 2-[4-(4-diméthylaminophényl)pipérazin-1-yl]4,5-dihydro-imidazo-[4,5,1-ij]quinoléin-6-one (R1 = R2 = R2' = H, R3 = -, R4 = para-diméthylaminophényle, X = N, n = m = 1) (composé n°58)

### 4.1: 1-(tert-butoxycarbonyl)-4-(4-diméthylaminophényl)pipérazine

On introduit successivement dans un ballon sous atmosphère d'azote muni d'un réfrigérant avec agitation magnétique, le 1-bromo-4-*N*,*N*-diméthylaniline (0.85 g, 4,25 mmoles), l'acétate de palladium (0,039 g, 0,17 mmole) le 2,2'-bis(diphényl-phosphino)-1,1'-binaphtyle (BINAP) (0,17 g, 0,25mmole) et le KOtBu (0,67 g, 5,95 mmoles). On purge le ballon 3 fois à l'azote, on introduit le toluène (25 ml) et la *N-tert-*butoxycarbonylpipérazine (0,95 g, 5,1 mmoles) et on chauffe le mélange réactionnel sous reflux pendant 20 heures. Après retour à température ambiante, on rajoute de l'acétate d'éthyle et lave la phase organique 2 fois à l'eau. Après séchage de la phase organique sur sulfate de sodium, filtration et concentration, on obtient un résidu (1,38 g) qui est chromatographié sur gel de silice avec un gradient de dichlorométhanelacétate d'éthyle: 9/1 à 1/1). Le composé du titre est obtenu avec un rendement de 50% (0,67 g). RMN ¹H (300 MHz, δ ppm) CDCl3: 1,47 (s, 9H), 2 ,99 (m, 4H), 3,57 (m, 4H), 6,72 (d, 2H), 6,89 (d, 2H).

### 4.2 : N-1-(4-diméthylaminophényl)pipérazine

Le produit décrit dans l'exemple 1.1 (0,67 g, 2,2 moles) est dissous dans de l'acide trifluoroacétique (acide trifluoroacétique, 20 ml) et gardé sous agitation magnétique pendant 2,5 heures à température ambiante. Après évaporation de l'acide trifluoroacétique, le résidu est repris par une solution saturée de carbonate de sodium et la phase aqueuse extraite 2 fois par de l'acétate d'éthyle. Les phases organiques rassemblées sont lavées à neutralité par une solution saturée de chlorure de sodium, séchées sur sulfate de sodium et concentrées. Après chromatographie du résidu sur gel de silice (dichlorométhane/méthanol/NH4OH : 95/5/0,1), le produit du titre est obtenu avec un rendement de 69% (0,31 g, Rf : 0,1).

### 4.3 : 2-[4-(4-diméthylaminophényl)-pipérazin-1-yl]4,5-dihydro-imidazo-[4,5,1-ij]quinoléin-6-one

On introduit successivement dans un ballon muni d'un réfrigérant avec agitation magnétique, l'intermédiaire chloré décrit au point 1.1 (0,2 g, 0,969 mmole), 1 ml de triéthylèneglycol monométhyl éther (TGME), la lutidine (0,125 ml, 1,07 mmole), le fluorure de césium (0,148 g, 0,969 mmole) et une solution du dérivé de l'exemple 1.2 (0,219 g, 1,07 mmole) dans le TGME (1 ml). On chauffe le mélange réactionnel à 140°C pendant 1,5 heures, laisse refroidir, rajoute de l'eau et extrait 2 fois à l'acétate d'éthyle. On lave les phases organiques 2 fois avec de l'eau, sèche sur sulfate de sodium, filtre et concentre. On chromatographie le résidu (0,38 g) sur gel de silice (acétate d'éthyle) et reprend l'huile obtenue (0,246 g) dans un peu de dichlorométhane et cristallise par addition d'éther. On filtre le solide blanc qui correspond au composé attendu (0,208 g, 57%). LC-MS: MH+ = 376. RMN ¹H (360 MHz, δ ppm) DMSO D6: 2,79 (s, 6H), 3,0 (t, 2H), 3,14 (m, 4H), 3,55 (m, 4H), 4,47 (t, 2H), 6,71 (d, 2H), 6,91 (d, 2H), 7,15 (dd, 1H), 7,35 (d, 1H), 7,60 (d, 1H).

### Exemple 5 : 2-[4-(4-tert-butoxycarbonylaminophényl)pipérazin-1-yl]-4,5-dihydro-imidazo[4,5,1-ij]quinoléin-6-one (R1 = R2 = R2' = H, R3 = -, X = N, R4 = 4-tert-butyloxycarbonylaminophényle, n = m = 1) (composé n° 57)

### 5.1 : 1-[2-(triméthylsilyl)éthylcarbonyl]-4-(4-nitrophényl)pipérazine

On maintient sous agitation magnétique sous reflux pendant 3 heures puis à température ambiante pendant 20 heures, une solution de 1-(4-nitrophényl)pipérazine (10 g, 48,31 mmoles) et de 2-(triméthylsilyl)éthyl-p-nitrophénylcarbonate (14,4 g, 50,73 mmoles) dans du tétrahydrofurane (220 ml). On concentre le mélange réactionnel, le reprend par du dichlorométhane et lave 4 fois la phase organique avec de la soude 1N et 2 fois avec de l'eau. La phase organique est ensuite séchée sur du sulfate de sodium, filtrée et concentrée ; le résidu huileux obtenu est utilisé tel quel dans l'étape suivante (12 g, rendement 71%). RMN ¹H (300 MHz, δ ppm) CDCl3: 0,04 (s, 9H), 1,02 (m, 2H), 3,43 (m, 4H), 3,65 (m, 4H), 4,22 (m, 2H), 6,82 (d, 2H), 8,13 (d, 2H).

### 5.2: 1-[2-(triméthylsilyl)éthylcarbonyl]-4-(4-aminophényl)pipérazine

On maintient sous atmosphère d'hydrogène, à pression ambiante, pendant 18 heures et sous forte agitation magnétique un mélange du dérivé nitré décrit précédemment (12 g, 34,2 mmoles) et de 4 à 5 spatules de Nickel de Raney dans 300 ml d'éthanol. Après filtration et concentration, le résidu obtenu est utilisé tel quel dans l'étape suivante : le produit attendu, présent à environ 50% d'après la RMN et la LC-MS, est difficile à purifier. RMN ¹H (300 MHz, δ ppm) CDCl3: 0,04 (s, 9H), 1,03 (m, 2H), 2,96 (m, 4H), 3,59 (m, 4H), 4,18 (m, 2H), protons aromatiques non attribuables, dû à la présence des impuretés.

### 5.3: 1-[2-(triméthylsilyl)éthylcarbonyl]-4-(4-tert-butoxycarbonyl-aminophényl) pipérazine

Dans un ballon muni d'un réfrigérant, on ajoute successivement le brut réactionnel de l'étape précédente (évalué à 31 mmoles) et du di-*tert*-butyl dicarbonate (7,5 g, 34,1 mmoles) dans du tétrahydrofurane (50 ml) et chauffe sous reflux pendant 44 heures. On évapore le tétrahydrofurane, reprend le résidu dans un mélange eau/acétate d'éthyle, lave 2 fois la phase organique à l'eau. La phase organique est concentrée, séchée sur sulfate de sodium, concentrée et le résidu chromatographié sur gel de silice (dichlorométhane/acétate d'éthyle : 1/1). Le produit obtenu impur, est utilisé tel quel pour l'étape suivante. LC-MS : MH+ = 422.

### 5.4: 1-(4-tert-butoxycarbonylaminophényl)pipérazine

On introduit successivement dans un ballon muni d'un réfrigérant avec agitation magnétique, l'intermédiaire décrit au point précédant (3 g, considéré à 7,12 mmoles) et le fluorure de tétrabutylammonium (3,37 g, 10,68 mmoles) dans du tatrahydrofurane (60 ml). On chauffe sous reflux pendant 2 heures, concentre le mélange réactionnel et reprend à l'eau et à l'acétate d'éthyle. On lave la phase organique 2 fois à l'eau, puis avec une solution saturée en chlorure de sodium, la sèche sur sulfate de sodium et la concentre. On obtient le composé du titre (0,99 g, 50% sur 3 étapes) après chromatographie sur gel de silice (dichlorométhane / méthanol / NH₄OH: 90/10/0,1). RMN ¹H (300 MHz, δ ppm) CDCl3: 1,50 (s, 9H), 3,04 (m, 8H), 6,60 (s, 1H), 6,87 (d, 2H), 7,23 (d, 2H).

### 5.5 : 2-[4-(4-tert-butoxycarbonylaminophényl)pipérazin-1-yl]-4,5-dihydro-imidazo[4,5,1-ij] quinoléin-6-one

On introduit successivement dans un ballon muni d'un réfrigérant avec agitation magnétique, l'intermédiaire chloré décrit au point 1.1. (0,7 g, 3,39 mmoles), du TGME (7 ml), la lutidine (0,43 ml, 3,73 mmoles), le fluorure de césium (0,514 g, 3,39 mmoles) et le 1-(4*-tert* butoxycarbonylaminophényl) pipérazine (1,03 g, 3,73 mmoles). On chauffe le mélange réactionnel à 140°C pendant 2 heures, laisse refroidir, rajoute de l'eau et extrait 2 fois à l'acétate d'éthyle. On lave les phases organiques 2 fois avec de l'eau, sèche sur sulfate de sodium, filtre et concentre. Une chromatographie du résidu sur gel de silice (gradient de dichlorométhane/acétate d'éthyle : 75/25 à 1/1) fournit le composé attendu qui est ensuite cristallisé dans un mélange dichlorométhane/éther pour donner un solide blanc (1,1 g, 73%). LC-MS: MH+ = 448. RMN ¹H (500 MHz, δ ppm) DMSO D6 1,45 (s, 9H), 3,00 (t, 2H), 3,22 (m, 4H), 3,55 (m, 4H), 4,47 (t, 2H), 6,91 (d, 2H), 7,15 (dd, 1H), 7,33 (m, 2H), 7,35 (d, 1H), 7,61 (d, 1H).

### Exemple 6 : 2-[4-(4-aminophényl)pipérazin-1-yl]4,5-dihydro-imidazo[4,5,1-ij] quinoléin-6-one, dichlorhydrate (R1 = R2 = R2' = H, R3 = -, R4 = para-aminophényle, X = N, n = m = 1) (composé n° 50)

On ajoute une solution d'acide chlorhydrique gaz dans de l'éthanol au composé de l'exemple 5 (0,22 g, 0,497 mmoles) et garde le mélange réactionnel sous agitation à température ambiante pendant 2 heures. On concentre et le composé attendu est obtenu après trituration dans l'éther sous la forme d'un solide blanc (0,155 g, 80%). RMN ¹H (500 MHz, δ ppm) DMSO D6: 3,06 (t, 2H), 3,63 (m, 4H), 3,84 (m, 4H), 4,65 (t, 2H), 7,10 (d, 2H), 7,26 (d, 2H), 7,36 (dd, 1H), 7,54 (d, 1H), 7,71 (d, 1H), 10,06 (pic large, 2H).

### Exemple 7 : 2-[4-(4-acétamidophényl)pipérazin-1yl]4,5-dihydro-imidazo [4,5,1-ij]quinoléin-6-one (R1 = R2 = R2' = H, R3 = -, R4 = para-acétamidophényle, X = N, n = m = 1) (composé n° 60)

On ajoute le composé de l'exemple 6 (0,20 g, 0,48 mmole) et la triéthylamine (80 µL, 0,572 mmole) à la solution d'acide acétique (27 µL, 0,468 mmole) et de carbonyldiimidazole (0,152 g, 0,936 mmole) dans la DMF (4 ml). On garde le mélange réactionnel sous agitation magnétique à 70°C pendant 20 heures. Après évaporation de la DMF sous vide, on reprend le résidu à l'eau, le triture et filtre le solide obtenu. Le lavage du solide avec un minimum de dichlorométhane puis avec de l'éther fournit le composé attendu (0,12 g, 64%) RMN ¹H (500 MHz, δ ppm) DMSO D6: 1,99 (s, 3H), 3,00 (t, 2H), 3,25 (m, 4H), 3,55 (m, 4H), 4,48 (t, 2H), 6,94 (d, 2H), 7,15 (dd, 1H), 7,35 (d, 1H), 7,44 (d, 2H), 7,61 (d, 1H), 9,70 (s, 1H).

### Exemple 8: 2-(4-n-propyl-pipérazin-1-yl)-4,5-dihydro-imidazo[4,5,1-ij] quinoléin-6-one, di-chlorhydrate (R1 = R2 = R2' = R4 = H, X = N, R3 = n-propyle, n = m = 1) (composé n° 26)

On introduit successivement dans un ballon muni d'un réfrigérant avec agitation magnétique, l'intermédiaire de formule (II) du point 1.1 (0,2 g, 0,969 mmole), 1 mL de triéthylèneglycol monométhyl éther (TGME), la lutidine (0,37 mL, 3,30 mmoles), le fluorure de césium (0,148 g, 0,969 mmole) et une solution de N-propyl-pipérazine, 2HBr (0,296 g, 1,18mmole) dans le TGME (1mL). On chauffe le mélange réactionnel à 140°C pendant 2 heures et 15 minutes, laisse refroidir, rajoute une solution saturée de carbonate de sodium et du chlorure de sodium solide et extraire 2 fois à l'acétate d'éthyle. On lave les phases organiques 2 fois avec une solution saturée de chlorure de sodium, sèche sur sulfate de sodium, filtre et concentre. On chromatographie le résidu obtenu sur gel de silice (dichtorométhane/méthanol/NH₄OH: 95/5/0.5) et synthétise le sel de di-chlorhydrate de l'huile obtenue (0,203 g) en la dissolvant dans un peu de dichlorométhane et en ajoutant une solution saturée d'acide chlorhydrique gaz dans de l'éther anhydre. On filtre le solide blanc qui correspond au composé attendu (0,23 g). LC-MS: MH+ = 299. RMN ¹H (360 MHz, δ ppm) DMSO D6: 0,94 (t, 3H), 1,78 (m, 2H), 3,08 (m, 4H), 3,28 (m, 2H), 3,62 (3, 2H), 3,88 (m, 2H), 4,25 (m, 2H), 4,65 (t, 2H), 7,42 (t, 1H), 7,60 (d, 1H), 7,76 (d, 1H), 11,66 (s, 1H)

### Exemple 9: 2-(4-tert-butyloxycarbonyl-pipérazin-1-yl)-4,5-dihydro- imidazo[4,5,1-ij]quinoléin-6-one (R1 = R2 = R2' = H, R3 = - , X = N, R4 = tert-butyloxycarbonyle, n = m = 1) (composé n° 12)

On introduit successivement dans un ballon muni d'un réfrigérant avec agitation magnétique, l'intermédiaire décrit au point 1.1 (0,25 g, 1.2mmole), du TGME (1,5 mL), la lutidine (0,155 mL, 1,33 mmoles), le fluorure de césium (0,184 g, 1,21 mmole) et une solution de *tert*-butyl-1-pipérazine carboxylate (0,237 g, 1,28 mmole) dans le TGME (1,5 mL). On chauffe le mélange réactionnel à 120°C pendant 3 heures, laisse refroidir, rajoute de l'eau et extrait 2 fois à l'acétate d'éthyle. On lave les phases organiques 2 fois avec de l'eau, sèche sur sulfate de sodium, filtre et concentre. On chromatographie le résidu obtenu sur gel de silice (dichlorométhane/acétate d'éthyle: 1/1) pour obtenir le composé attendu sous forme d'un solide blanc (0,315g, 73%). LC-MS: MH+ = 357. RMN ¹H (500 MHz, δ ppm) DMSO D6: 1,51 (s, 9H), 3,07 (t, 2H), 3,46 (m, 4H), 3,58 (m, 4H), 4,52 (t, 2H), 7,23 (t, 1H), 7,43 (d, 1H), 7,68 (d, 1H)

### Exemple 10 : 2-(pipérazin-1 yl)-4,5-dihydro-imidazo[4,5,1-ij]quinoléin-6-one, di-chlorhydrate (R1 = R2 = R2' = R3 = R4 = H, X = N, n = m = 1) (composé n° 13)

On ajoute une solution saturée d'acide chlorhydrique gaz dans de l'éther anhydre au composé de l'exemple 9 (0,1 g, 0,28 mmole) préalablement dissout dans un minimum de méthanol et garde sous agitation magnétique à température ambiante pendant la nuit. On concentre et cristallise le résidu dans un mélange méthanol/dichlorométhane. Le composé attendu est obtenu sous forme d'un solide blanc (0,07g). LC-MS: MH+ = 257. RMN ¹H (500 MHz, δ ppm) DMSO D6: 3,12 (t, 2H), 3,95 (m, 4H), 3,94 (m, 4H), 4,66 (t, 2H), 7,43 (t, 1H), 7,61 (d, 1H), 7,80 (d, 1H), 9,63 (s, 2H).

### Exemple 11 : 2-(4-acétyl-pipérazin-1-yl)-4,5-dihydro-imidazo[4,5,1-ij] quinoléin-6-one, di-chlorhydrate (R1 = R2 = R2' = H, R3 = -, R4 = COCH₃, X = N, n = m = 1) (composé n° 15)

On ajoute le composé de l'exemple 10 (0,085 g, 0,26 mmole) et la triéthylamine (75 µL, 0,52 mmole) à la solution d'acide acétique (20 µL, 0,349 mmole), d'hydroxybenzotriazole (0,055 g, 0,349 mmole) et de diisopropyl azodicarboxylate (55 µL, 0,349 mmole) dans le dichlorométhane (5 mL). On garde le mélange réactionnel sous agitation magnétique à température ambiante pendant la nuit et le dilue avec de l'acétate d'éthyle et une solution d'acide chlorhydrique 1N. L'obtention du produit brut se fait par extraction acido-basique et lavages aqueux usuels suivis de séchage des phases organiques sur sulfate de sodium et évaporation. Une chromatographie sur gel de silice (dichlorométhane/éthanol: 95/5) fournit le composé attendu (0,04 g). LC-MS: MH+ = 299. RMN ¹H (500 MHz, δ ppm) DMSO D6: 2,13 (s, 3H), 3,08 (t, 2H), 3,46 (m, 2H), 3,52 (m, 2H), 3,70 (m, 4H), 4,54 (t, 2H), 7,23 (t, 1H) 7,43 (d, 1H), 7,68 (d, 1H).

### Exemple 12 : 2-[4-(4-pyridyl)-pipérazin-1-yl)-4,5-dihydro-imidazo[4,5,1-ij]quinoléin-6-one, di-chlorhydrate (R1 = R2 = R2' = H, R3 = -, R4 = 4-pyridyle, X = N, n = m = 1) (composé n° 23)

On introduit successivement dans un ballon muni d'un réfrigérant avec agitation magnétique, l'intermédiaire décrit au point 1.1 (0,2 g, 0,969 mmole), du TGME (1mL), la lutidine (125 µL, 0,969 mmoles), le fluorure de césium (0,148 g, 0,969 mmole) et une solution de 1-(4-pyridyl)pipérazine (0,238 g, 1,02 mmole) dans le TGME (1mL). On chauffe le mélange réactionnel à 140°C pendant 1heure et 50 minutes, laisse refroidir, rajoute une solution saturée de carbonate de sodium et du chlorure de sodium solide et extrait 2 fois à l'acétate d'éthyle. On lave les phases organiques 2 fois avec une solution saturée de chlorure de sodium, sèche sur sulfate de sodium, filtre et concentre. On chromatographie le résidu obtenu sur gel de silice (dichlorométhane/méthanol/NH₄OH: 95/5/0.5) pour obtenir le composé attendu sous forme d'un solide blanc (0,071 g). LC-MS: MH+ = 334. RMN ¹H (360 MHz, δ ppm) DMSO D6: 3,01 (t, 2H), 3,58 (m, 8H), 4,51 (t, 2H), 6,91 (d, 2H), 7,18 (t, 2H), 7,37 (d, 1H), 7,63 (d, 1H), 8,20 (d, 2H)

### Exemple 13 : 2-(4-benzyl-pipéridin-1-yl)-4,5-dihydro-imidazo[4,5,1-ij] quinoléin-6-one, di-chlorhydrate (R1 = R2 = R2' = R3 = H, R4 = benzyle, X = C, n = m = 1) (composé n° 1)

On introduit successivement dans un ballon muni d'un réfrigérant avec agitation magnétique et sous atmosphère d'azote, l'intermédiaire décrit au point 1.1 (0,206 g, 1 mmole) en solution dans du toluène anhydre (8 mL), le *tert*-butylate de potassium (0,157 mg, 1,4 mmole), le 2,2'-bis(diphénylphosphino)-1,1'-binaphtyle (0,04 g, 0,06 mmole), l'acétate de palladium (II) (0,009 g, 0,04 mmole) et une solution de N-benzylpipérazine (0,21 g, 1,2 mmole) dans du toluène (2 mL). On chauffe le mélange réactionnel à 85°C pendant la nuit, laisse refroidir, dilue à l'acétate d'éthyle et lave successivement à l'eau (3 fois) et avec une solution saturée de chlorure de sodium, sèche sur sulfate de sodium, filtre et concentre. On chromatographie le résidu (0,24 g, dichlorométhane/ acétate d'éthyle: 8/2) pour obtenir le produit attendu (0,075 g). LC-MS: MH+ = 346. RMN ¹H (360 MHz, δ ppm) DMSO D6: 1,37 (m, 1H), 1,67 (d large, 1H), 1,80 (m, 1H), 2,58 (d, 1H), 2,84-3,00 (t, 2H et m, 1H), 3,81 (d large, 1H), 4,40 (t, 2H), 7,12 (t, 1H), 7,20 (m, 3H), 7,26-7,37 (m, 1H et d, 1H), 7,5 (d,1H)

### Exemple 14: 1-(6-oxo-5,6-dihydro-4H-imidazo[4,5,1-ij]quinoléin-2-yl)-4-phényl-4-pipéridinecarboxamide (R1 = R2 = R2' = H, R3 = CONH₂, R4 = phényle, X = C, n = m = 1) (composé n° 43)

### 14.1 : Composé de formule (III) : 4-phénylpipéridine-4-carboxamide.

Le composé est obtenu selon la synthèse décrite dans la littérature : *Bioorg*. *Med*. *Chem. Lett.* **7** (19),1997, 2531-2536.

### 14.2 : 1-(6-oxo-5,6-dihydro-4H-imidazo[4,5,1-ij]quinoléin-2-yl)-4-phényl-4-pipéridinecarboxamide.

Dans un tricol de 50 ml, sous azote, on introduit 300 mg de composé (II), décrit au point 1.1, 5 ml de diméthylformamide , 70 mg de fluorure de potassium, 0,2ml de 2,6 lutidine, et 510 mg de composé (IV), décrit ci-dessus. Le mélange est agité 4 heures à 140°C, et évaporé ensuite sous vide poussé. Le résidu obtenu est chromatographié sur gel de silice (70-200 microns) avec un mélange de 2% de méthanol dans le dichlorométhane. Le produit obtenu est ensuite recristallisé dans un minimum d'acétone à chaud. On obtient 270 mg de cristaux blancs. Les signaux du spectre RMN correspondent au produit attendu : RMN¹H (200 MHz, δ ppm) DMSO D6: 2,0 (m, 2H), 2,5 (m, 2H), 3,0 (t, 2H), 3,2-3,8 (m, 4H), 4,45 (t, 2H), 7,0-7,5 (m, 10H)

### Exemple 15 : 1-(6-oxo-5,6-dihydro-4H-imidazo[4,5,1-ij]quinoléin-2-yl)-4-phényl-4-aminopipéridine, dichlorhydrate (R1 = R2 = R2' = H, R3 = NH₂, R4 = phényle, X = C, n = m = 1) (composé n° 46)

On ajoute 4 ml de solution aqueuse d'acide chlorhydrique concentré au composé 42 du tableau (0,53 g, 1,21 mmole), fabriqué selon les méthodes décrites ci-dessus, préalablement dissout dans 5 ml de méthanol et garde sous agitation magnétique à température ambiante pendant 30 minuntes. On concentre sous vide et reprend le résidu dans l'éthanol absolu puis re-évapore à sec. Le composé attendu est obtenu sous forme d'un solide blanc (0,45 g). RMN ¹H (200 MHz, δ ppm) DMSO D6: 2,4-2,8 (m, 4H), 3,1 (t, 2H), 3,7 (m, 2H), 4,2 (m, 2H), 4,8 (t, 2H), 7,4-7,8 (m, 8H), 9,0 (s, 2H).

### Exemple 16: 2-[4-(4-hydroxyphényl)-pipérazin-1-yl]-4,5-dihydro-imidazo [4,5,1-ij]quinoléin-6-one (R1 = R2 = R2' = H, R3 = - , X = N, R4 = 4-hydroxyphényle, n = m = 1) (composé n° 71)

### 16.1. 2-chloro-4,5-dihydro-imidazo[4,5,1-ij]quinoléin-6-one, éthylène cétal

On introduit successivement, dans un ballon muni d'un réfrigérant avec agitation magnétique, une solution de l'intermédiaire décrit au point 1.1 (0,6 g, 2,9 mmoles) dans du 1,2-dichloroéthane (10 mL), de l'éthylène glycol (0,216 g, 3,49 mmoles), de l'acide p-toluène sulfonique (0,01 g), du triméthylorthoformate (0,372 g, 3,49 mmoles) et on complète avec 13 mL de 1,2-dichloroéthane. On chauffe sous reflux pendant 24 heures, rajoute les mêmes quantités des réactifs (éthylène glycol, acide p-toluène sulfonique, triméthylorthoformate) et on chauffe pendant encore 18 heures. Après évaporation du solvant, on reprend à l'acétate d'éthyle, lave avec une solution diluée de carbonate de sodium puis avec de l'eau, sèche la phase organique et tire à sec. On chromatographie le résidu sur gel de silice (gradient dichlorométhane pur à dichlorométhane / acétate d'éthyle : 96 / 4) pour obtenir le composé du titre (0,54 g, 74%). MH+ = 251.

### 16.2. Préparation du 2-[4-(4-benzyloxyphényl)-pipérazin-1-yl]-4,5-dihydro-imidazo[4,5,1-ij]quinoléin-6-one, éthylène cétal

On introduit successivement, dans un ballon muni d'un réfrigérant avec agitation magnétique, l'intermédiaire décrit au point 16.1. (0,54 g, 2,16 mmoles), du TGME (3 mL), la lutidine (277 µL, 2,38 mmoles), le fluorure de césium (0,328 g, 2,16 mmoles) et du 1-(4-benzyloxyphényl)pipérazine (0,725 g, 2,38 mmoles). On chauffe le mélange réactionnel à 100°C pendant 3 heures, rajoute de la lutidine (300 µL, 2,57 mmoles) et chauffe à 120°C pendant 8 heures. On laisse refroidir, rajoute de l'eau et extrait 2 fois à l'acétate d'éthyle. On lave les phases organiques 2 fois avec de l'eau, sèche sur sulfate de sodium, filtre et concentre. On purifie le résidu obtenu par 2 chromatographies sur gel de silice (gradient de dichlorométhane / acétate d'éthyle: 90 /10 à dichlorométhane /acétate d'éthyle: 40 / 60, puis gradient d'acétate d'éthyle à acétate d'éthyle / méthanol : 95 / 5) pour obtenir le composé attendu contaminé par environ 50% de cétone provenant de l'hydrolyse de la fonction cétal (0,365 g). LC-MS: MH+ = 483, MH+ = 439.

### 16.3. Préparation du 2-[4-(4-hydroxyphényl)pipérazin-1-yl]-4,5-dihydro-imidazo[4,5,1-ij]quinoléin-6-one, éthylène cétal

On introduit dans un ballon le mélange de composés de l'exemple 16.2. (0,365g), de l'éthanol (60 mL), du dichlorométhane (environ 0,5 mL) et du palladium sur charbon à 10% (0,04 g) et on garde sous agitation magnétique et sous atmosphère d'hydrogène pendant 17 heures à température ambiante. Après filtration et évaporation du solvant, on chromatographie le résidu sur gel de silice (gradient de dichlorométhane à dichlorométhane / méthanol: 94 / 6) pour obtenir le produit du titre (0,132 g). RMN ¹H (300 MHz, δ ppm) DMSO D6: 2,2 (t, 2H), 3,13 (m, 4H), 3,46 (m, 3H), 4,08-4,20 (m, 4H), 4,21 (t, 2H), 6,68 (dd, 2H), 6,86 (dd, 2H), 7,04 (m, 2H), 7,32 (m, 1H), 8,91 (s, 1H).

### 16.4. Préparation du 2-[4-(4-hydroxyphényl)-pipérazin-1-yl]-4,5-dihydro-imidazo [4,5,1-ij]quinoléin-6-one

On dissout le 2-[4-(4-hydroxyphényl)-pipérazin-1-yl]-4,5-dihydro-imidazo[4,5,1-ij] quinoléin-6-one, éthylène cétal (0,055 g, 0,14 mmole) dans 11 mL de tétrahydrofurane et on ajoute 3,6 mL d'une solution aqueuse d'acide chlorhydrique à 5%. On laisse le mélange sous agitation magnétique pendant 16 heures à température ambiante, puis à 50°C pendant 2 heures. Après dilution avec de l'eau, on alcalinise avec du bicarbonate de sodium, on extrait 2 fois à l'acétate d'éthyle et on sèche les phases organiques sur sulfate de sodium. Après traitement usuel, on purifie le résidu par chromatographie sur gel de silice (gradient de dichlorométhane à dichlorométhane / méthanol : 94 / 6) pour obtenir le produit du titre (0.027 g, 55%). RMN ¹H (500 MHz, δ ppm) DMSO D6: 2,99 (t,2H), 3,13 (m,4H), 3,53 (m,4H), 4,47(t,2H), 6,67 (d,2H), 6,85 (d,2H), 7,15 (t,1H), 7,34 (d,1H), 7,60 (d, 1H), 8,88 (s, 1H).

### Exemple 17 : 2-[4-(4-fluorophényl)-pipérazin-1-yl]-4,5-dihydro-9-méthyl-imidazo[4,5,1-ij]quinoléin-6-one (R1= 9-CH₃, R2 = R2' = H, R3 = - , X = N, R4 = 4-fluorophényle, n = m = 1) (composé n° 84)

On introduit successivement, dans un ballon muni d'un réfrigérant avec agitation magnétique, l'intermédiaire décrit au point 1.4 (0,2 g, 0,907 mmole), du TGME (2 mL), la lutidine (0,116 mL, 0.998 mmoles), le fluorure de césium (0,138 g, 0,907 mmole) et la 4-fluorophényl-pipérazine (0,180 g, 0,998 mmole). On chauffe le mélange réactionnel à 140°C pendant 3 heures, laisse refroidir, rajoute de l'eau et extrait 2 fois à l'acétate d'éthyle. On lave les phases organiques 2 fois avec de l'eau, sèche sur sulfate de sodium, filtre et concentre. On chromatographie le résidu obtenu sur gel de silice (gradient de dichlorométhane pur à dichlorométhane / méthanol : 98 / 2) pour obtenir le composé attendu sous forme d'un solide blanc (0,206 g, 62%). RMN ¹H (500 MHz, δ ppm) DMSO D6: 2,50 (s,3H), 2,97 (t,2H), 3,28 (m,4H), 3,54 (m,4H), 4,45 (t,2H), 7,05 (m,5H), 7,29 (d,1H).

### Exemple 18 : 1-[4-(4-fluorophényl)-pipérazin-1-yl]-3-méthyl-8,9-dihydro-7H-2,9a-diaza-benzo[cd]azulen-6-one (R1 = 3-CH₃, R2 = R2' = H, R3 = - , X = N, R4 = 4-fluorophényle, n = 2, m = 1) (composé n° 85)

On introduit successivement, dans un ballon muni d'un réfrigérant avec agitation magnétique, le 1-chloro-3-méthyl-8,9-dihydro-7*H*-2,9a-diaza-benzo[cd] azulen-6-one (0,2 g, 0,852 mmole), du TGME (2 mL), la lutidine (0,109 mL, 0,937 mmoles), le fluorure de césium (0,130 g, 0,852 mmole) et la 4-fluorophényl-pipérazine (0,169 g, 0,937 mmole). On chauffe le mélange réactionnel à 140°C pendant 3,5 heures, laisse refroidir, rajoute de l'eau et extrait 2 fois à l'acétate d'éthyle. Après traitement usuel, on chromatographie le résidu obtenu sur gel de silice (gradient de dichlorométhane pur à dichlorométhane /acétate d'éthyle: 60 / 40) pour obtenir le composé du titre sous forme d'un solide blanc (0,137g, 42%). RMN ¹H (500 MHz, δ ppm) DMSO D6: 2,20 (m,2H), 2,53 (s,3H), 3,04 (m,2H), 3,30 (m,4H), 3,40 (m,4H), 4,25 (m,2H), 7,07 (m,5H), 7,61 (d,1H).

### Exemple 19 : 2-[4-(4-fluorophényl)-pipérazin-1-yl]-4,5-dihydro-7-méthyl- imidazo[4,5,1-ij]quinoléin-6-one (R1= 7-CH₃, R2 = R2' = H, R3 = - , X = N, R4 = 4-fluorophényle, n = m = 1) (composé n° 83)

On introduit successivement, dans un ballon muni d'un réfrigérant avec agitation magnétique, le 2-chloro-4,5-dihydro-7-méthyl-imidazo[4,5,1-ij]quinoléin-6-one (0,245 g, 1,1 mmole), du TGME (2,5 mL), la lutidine (0,161 mL, 1,21 mmoles), le fluorure de césium (0,167 g, 1,1 mmole) et la 4-fluorophényl-pipérazine (0,219 g, 1,1 mmole). On chauffe le mélange réactionnel à 140°C pendant 2,5 heures, laisse refroidir, rajoute de l'eau et extrait 2 fois à l'acétate d'éthyle. Après traitement usuel, on chromatographie le résidu obtenu sur gel de silice (gradient de dichlorométhane/ acétate d'éthyle : 80 / 20 à dichlorométhane / acétate d'éthyle : 40 / 60) pour obtenir le composé du titre sous forme d'un solide blanc (0,26 g, 65%). RMN ¹H (500 MHz, δ ppm) DMSO D6: 2,59 (s,3H), 2,98 (t,2H), 3,26 (m,4H), 3,49 (m,4H), 4,42 (t,2H), 6,94 (d,1H), 7,05 (m,4H), 7,50 (d,1H).

### Exemple 20 : 2-[4-(4-fluorophényl)-pipérazin-1-yl]-4,5-dihydro-8-méthyl- imidazo[4,5,1-ij]quinoléin-6-one (R1= 8-CH₃, R2 = R2' = H, R3 = - , X = N, R4 = 4-fluorophényle, n = m = 1) (composé n° 82)

Le composé du titre est obtenu à partir d'un mélange 1 / 2 de 2-chloro-4,5-dihydro-8-méthyf-imidazo[4,5,1-ij]quinoiéin-6-one et de 2-chloro-4,5-dihydro-7-méthyl-imidazo[4,5,1-ij]quinoléin-6-one en utilisant les conditions décrites dans l'exemple 19. Après chromatographie sur gel de silice, 2 cristallisations successives ont permis d'obtenir l'isomère désiré contaminé par 4% seulement de l'autre isomère (0,053 g, rendement calculé sur le bon isomère : 48%). RMN ¹H (500 MHz, δ ppm) DMSO D6: 2,40 (s,3H), 2,98 (t,2H), 3,26 (m,4H), 3,55 (m,4H), 4,45 (t,2H), 7,06 (m,4H), 7,18 (s,1H), 7,44 (s,1H).

### Exemple 21 : 2-[4-(4-fluorophényl)-pipérazin-1-y1]-4,5-dihydro 7-méthoxy- imidazo[4,5,1-ij]quinoléin-6-one (R1= 7-OCH₃, R2 = R2' = H, R3 = - , X = N, R4 = 4-fluorophényle, n = m = 1) (composé n° 81)

Le composé du titre est obtenu à partir du 2-chloro-pipérazin-1-yl-4,5-dihydro-7-méthoxy-imidazo[4,5,1-ij]quinoléin-6-one en utilisant les conditions décrites dans l'exemple 19 (29%). RMN ¹H (500 MHz, δ ppm) DMSO D6: 2,89 (t,2H), 3,26 (m,4H), 3,44 (m,4H), 3,81 (s,3H), 4,36 (t,2H), 6,79 (d,1H), 7,06 (m,4H), 7,58 (d,1H).

Le tableau qui suit illustre les structures chimiques et les propriétés physiques de quelques composés de l'invention répondant à la formule générale (I). Selon que n = 1 ou 2 dans cette formule, deux composés de formule (I) ont été représentés ci-après avec, pour chacun d'eux, la numérotation des atomes du noyau benzimidazole.

Dans ce tableau :
- HCl représente un chlorhydrate et CF₃CO₂H représente un sel de trifluoroacétate, alors que « - » représente un composé sous forme libre,
- Me, Et, n-Pr et tBu représentent respectivement des groupes méthyle, éthyle, n-propyle et tert-butyle,
- Bn et Ph représentent respectivement des groupes benzyle et phényle,
- sauf mention contraire, les analyses RMN correspondent à des RMN du proton et les mesures sont effectuées dans du DMSO D6. * et ** signifient que les mesures sont effectuées, respectivement, à 360 MHz et à 500 MHz. Si aucune indication de ce genre n'est reportée, alors la mesure est effectuée à 200 MHz.

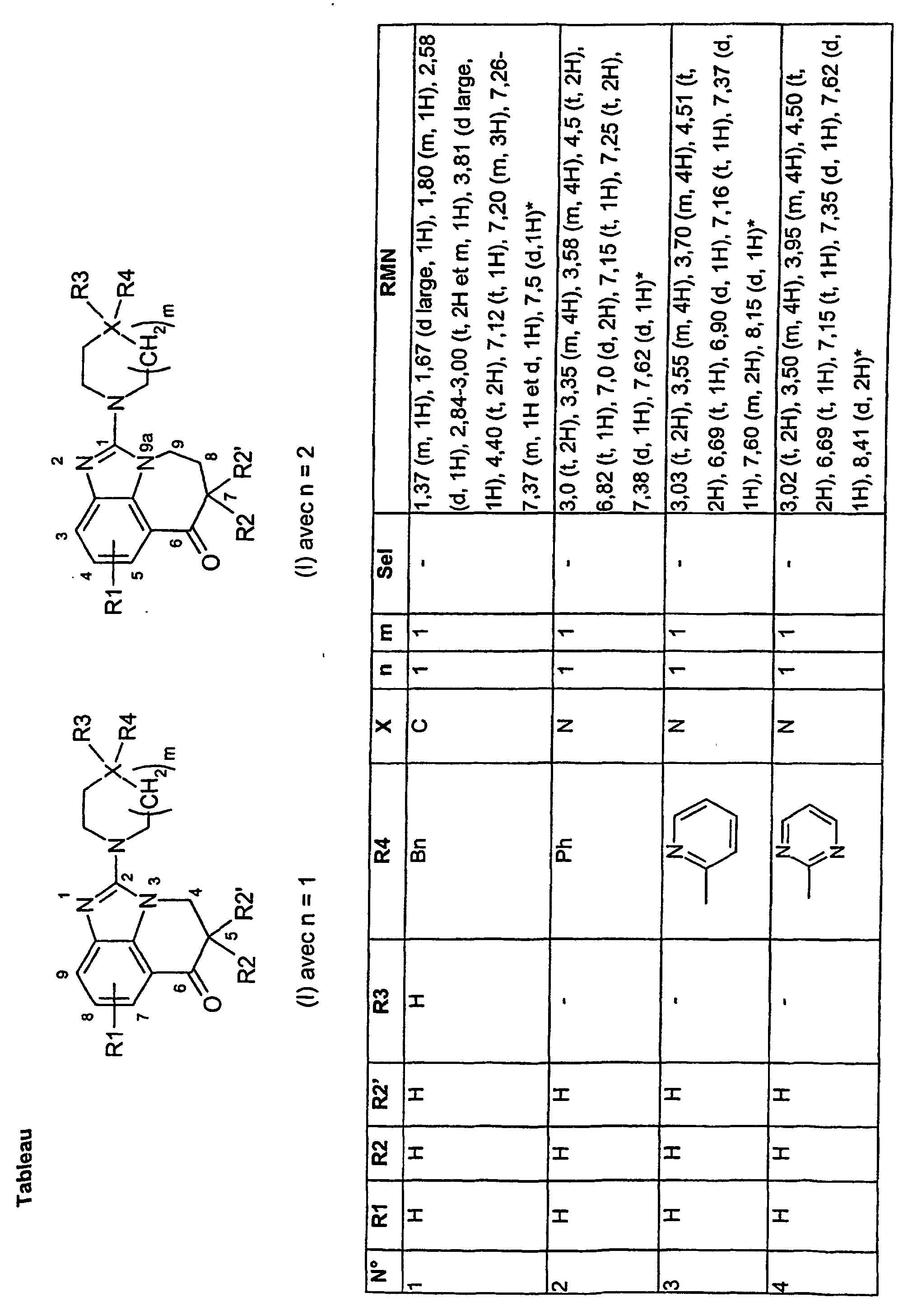

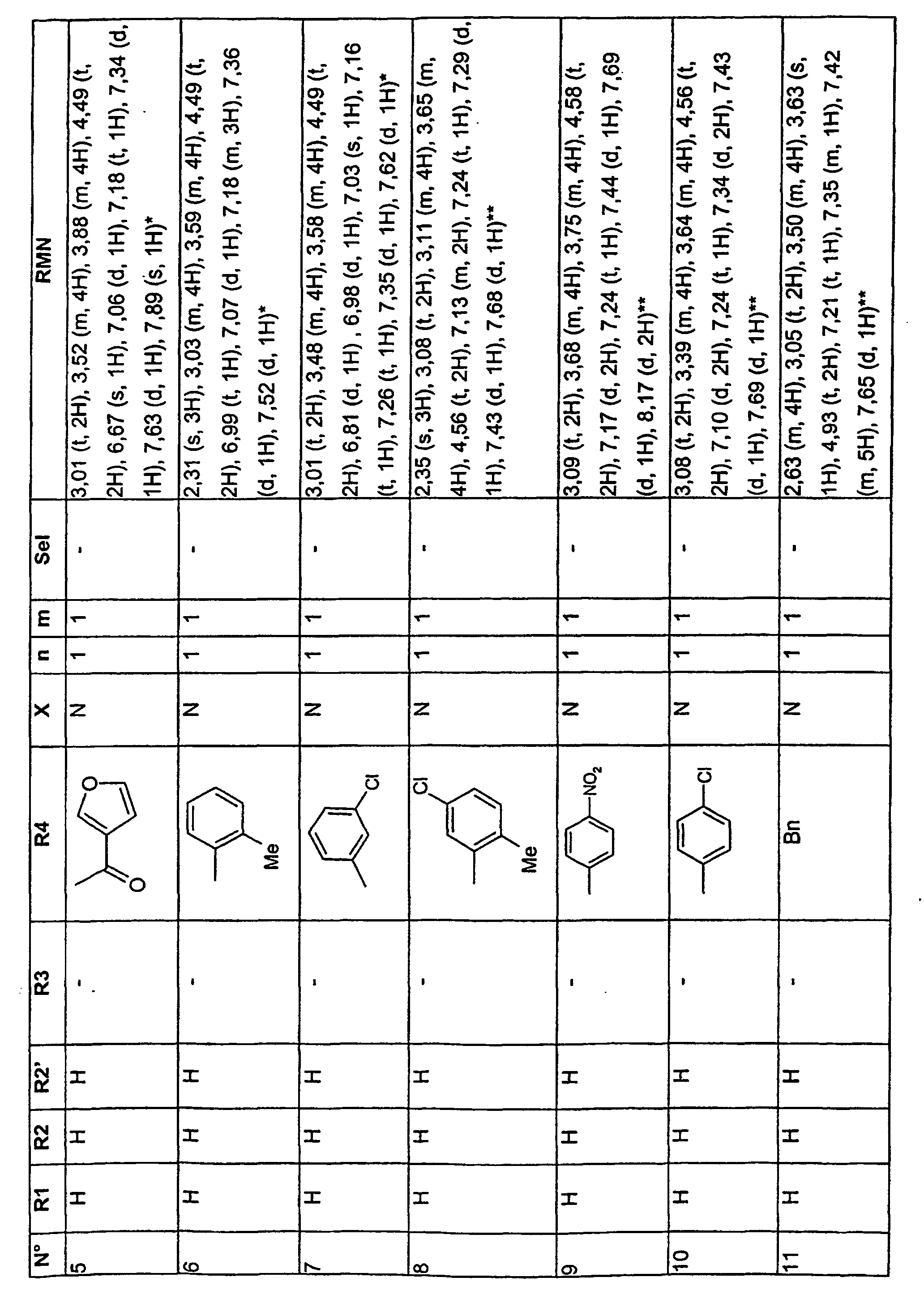

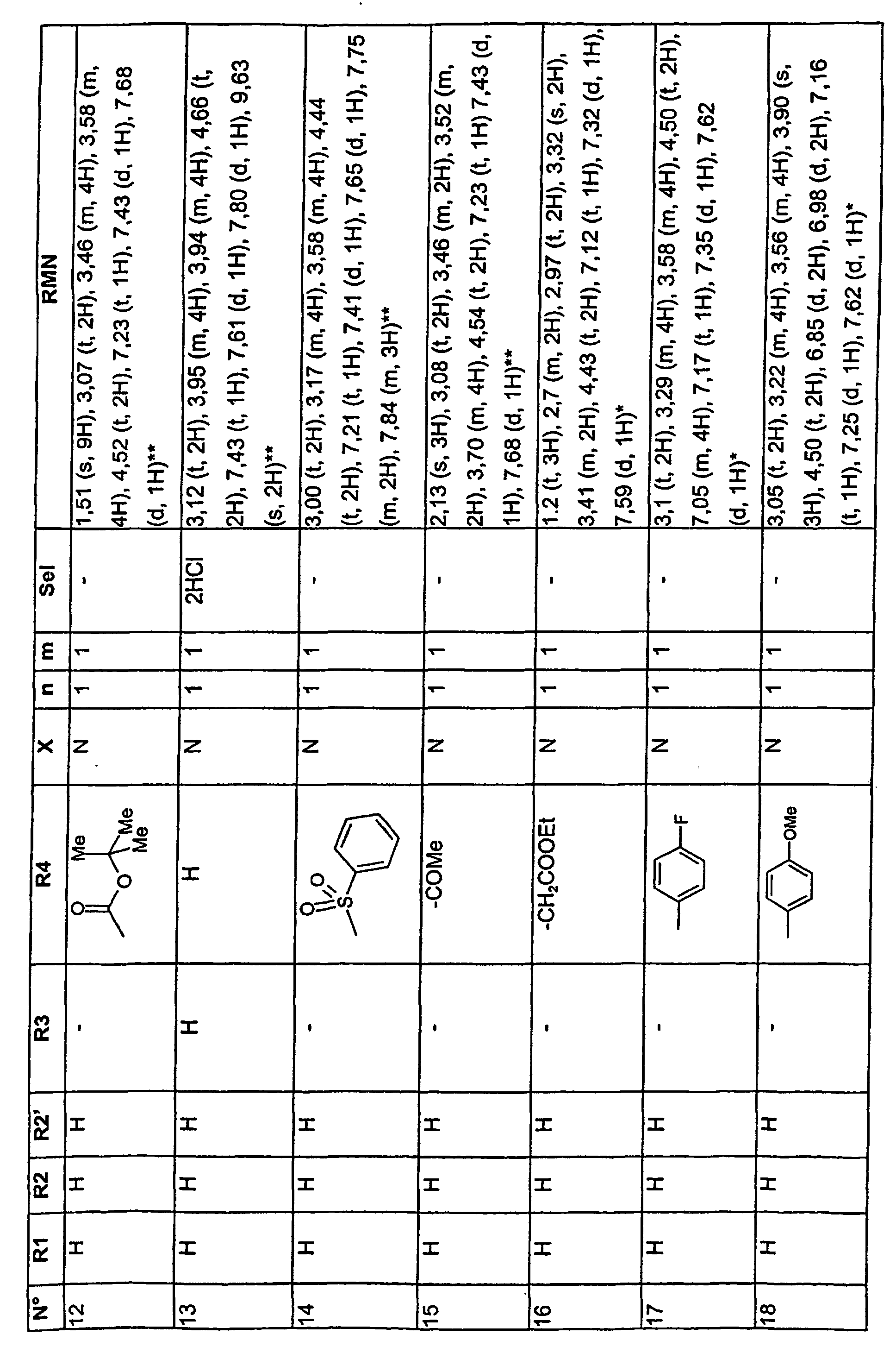

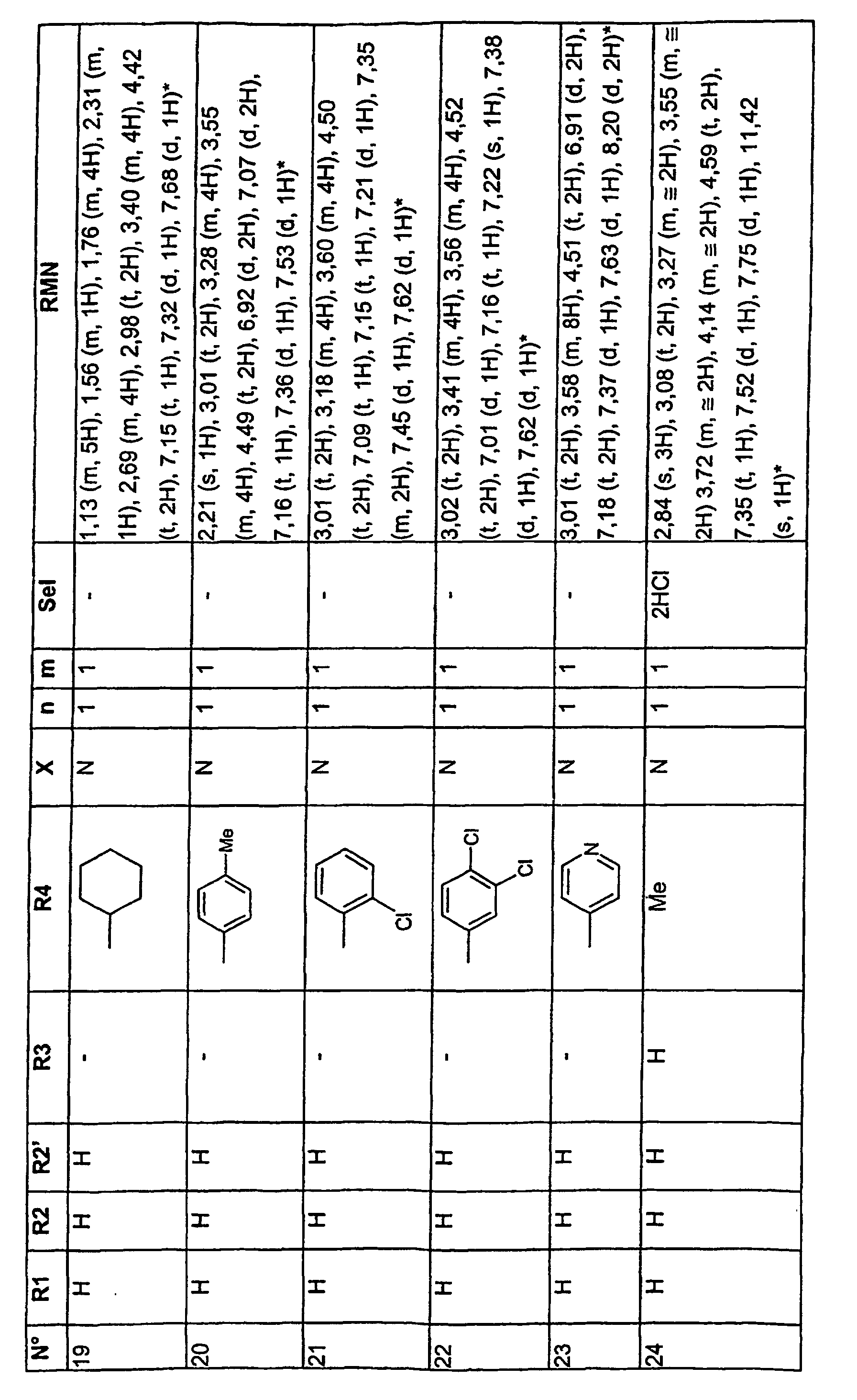

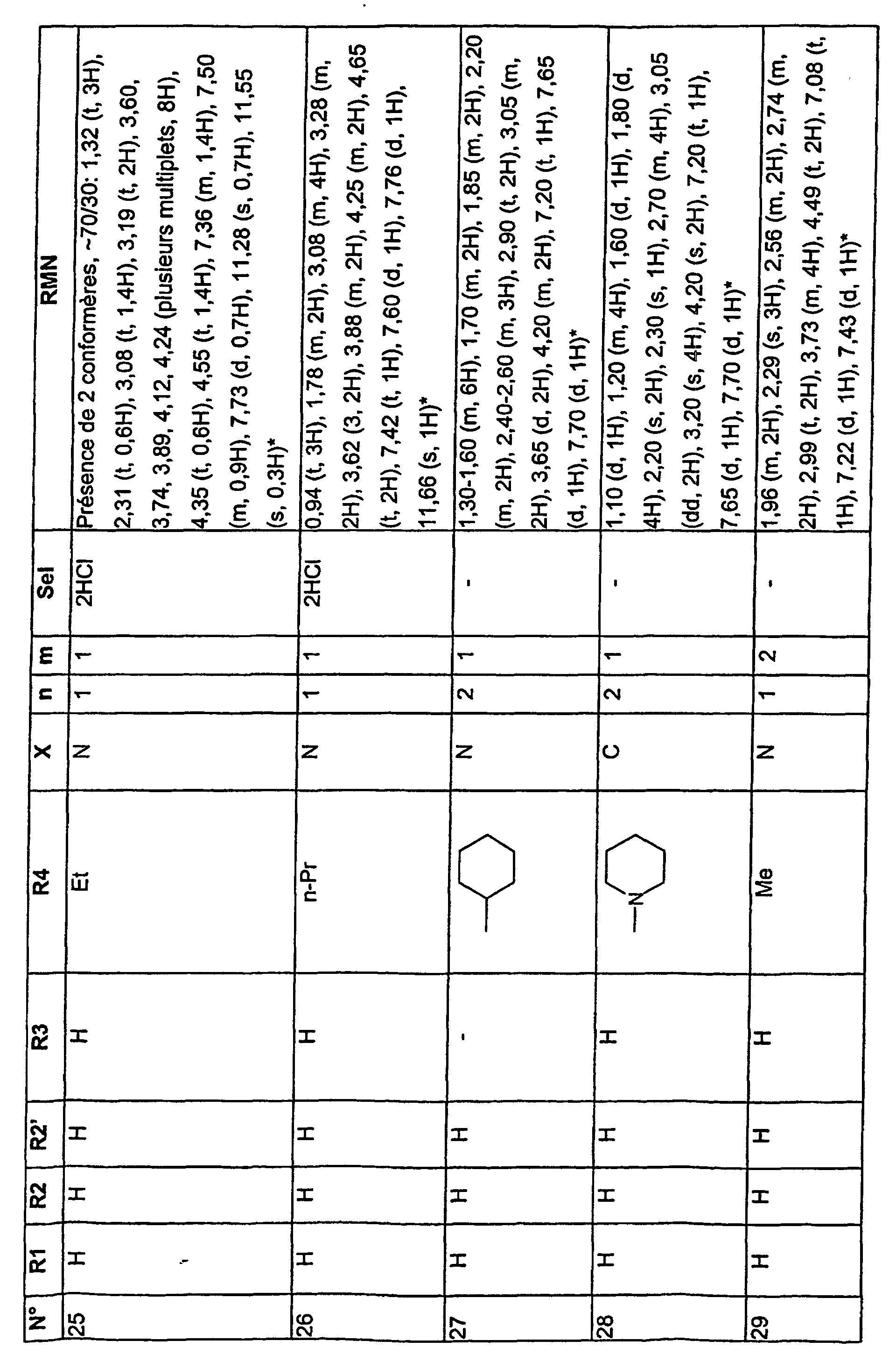

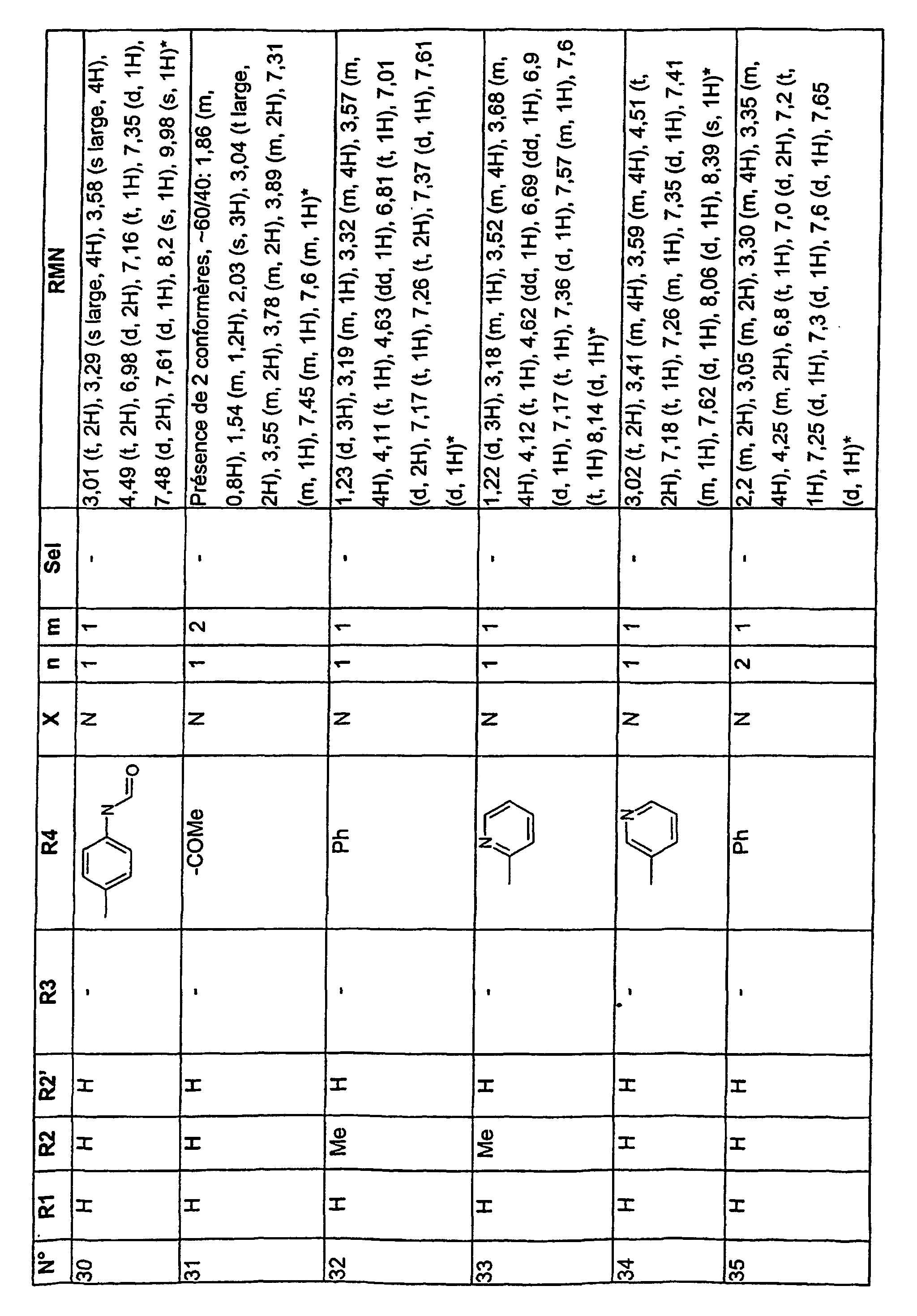

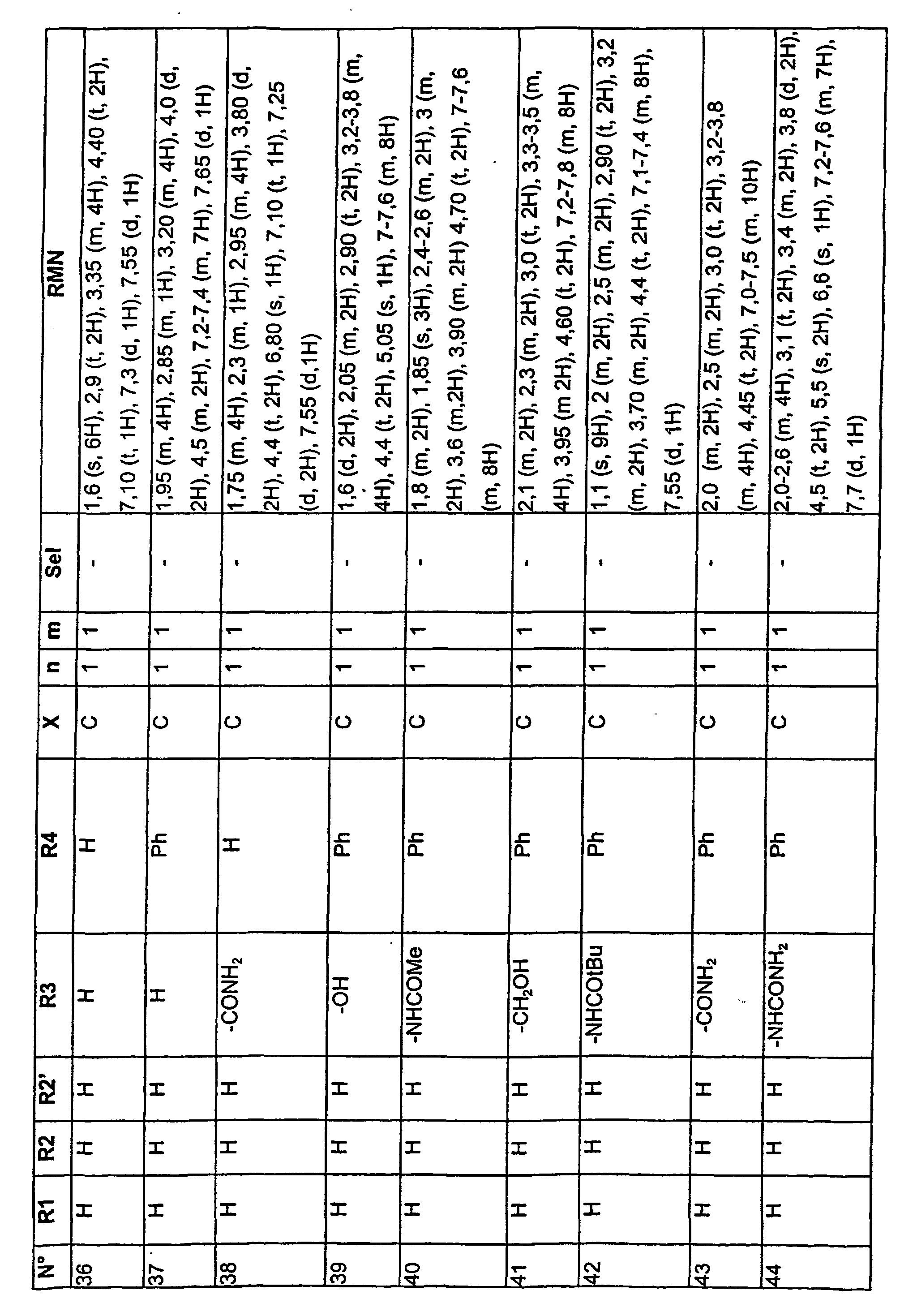

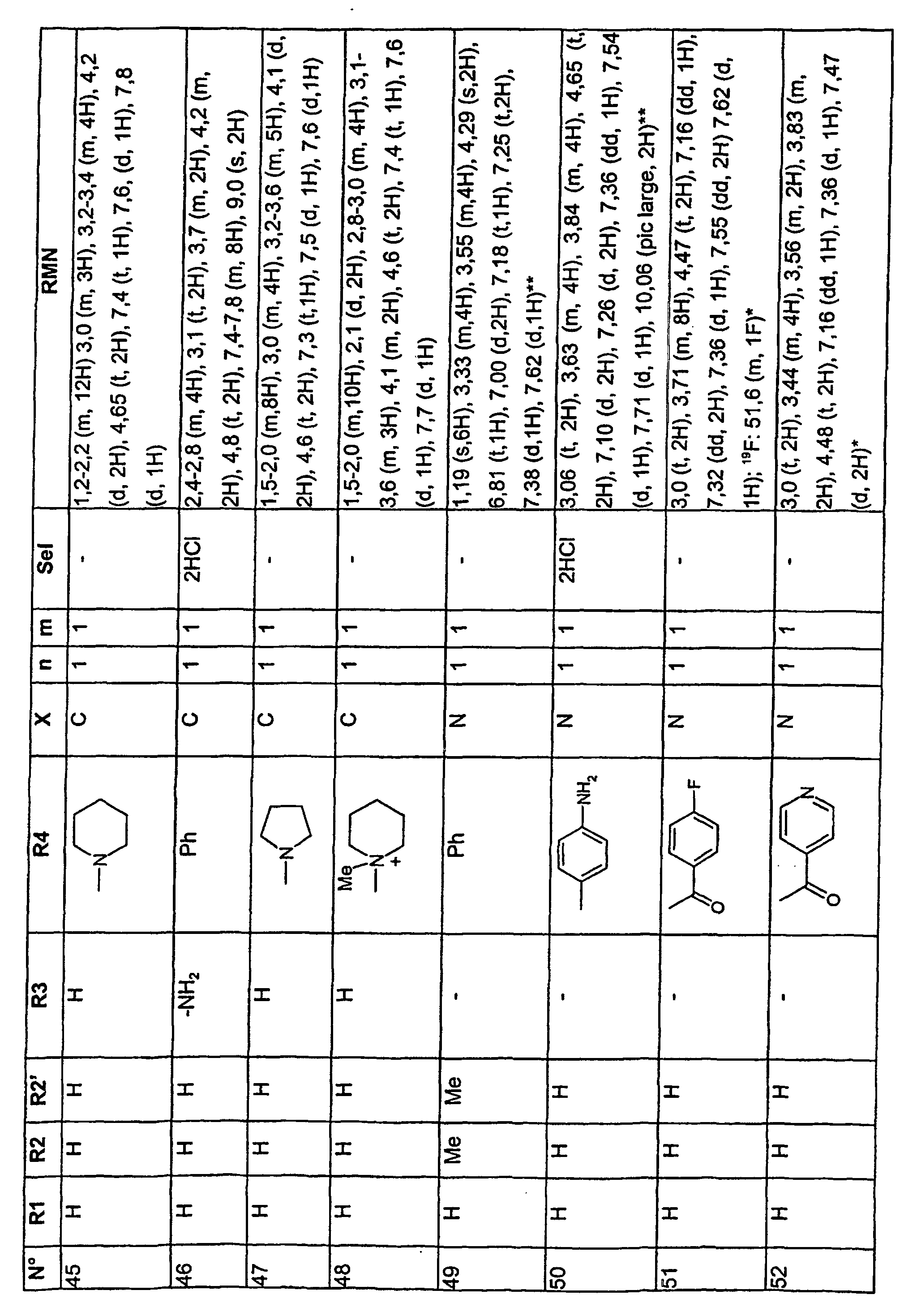

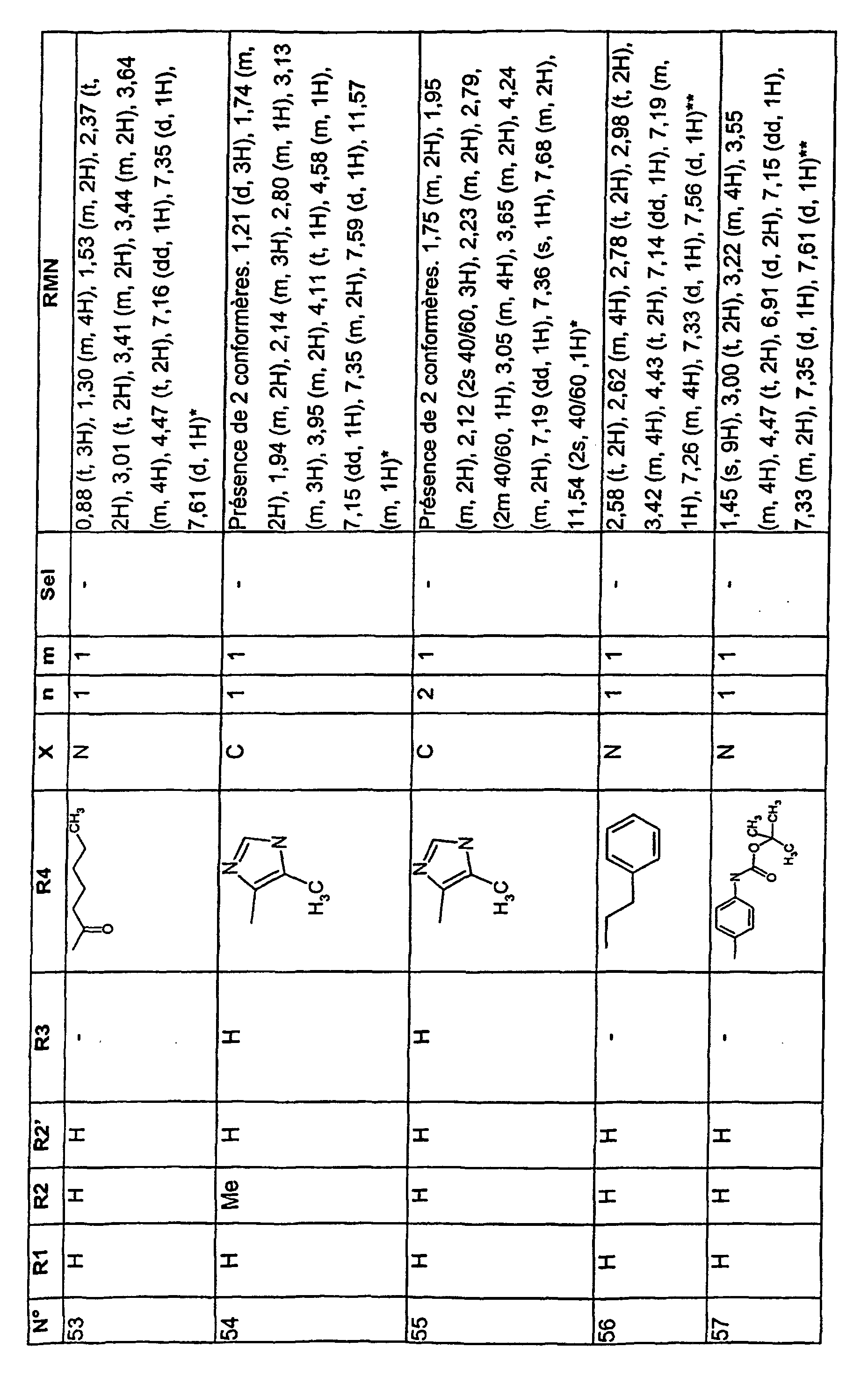

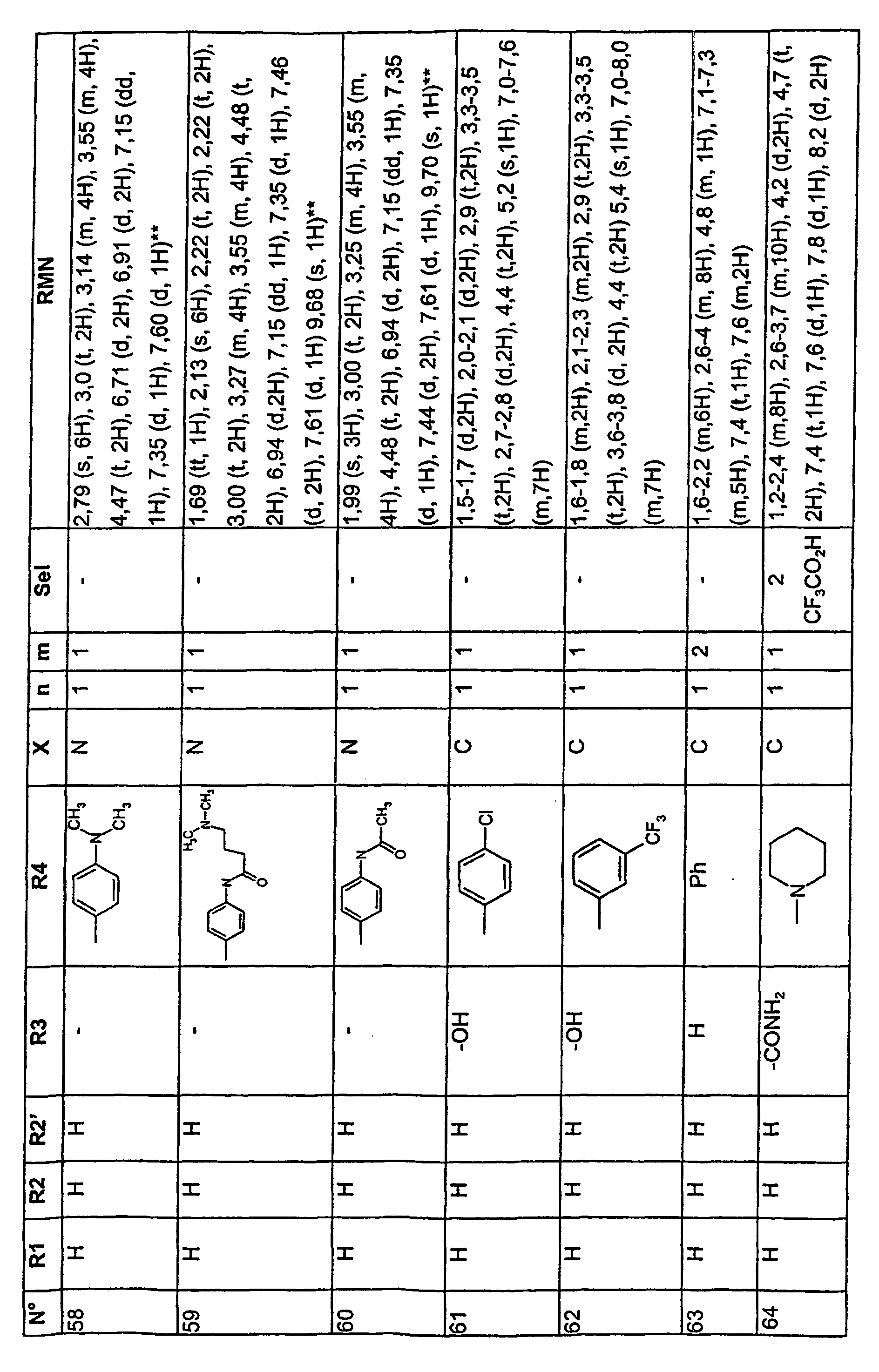

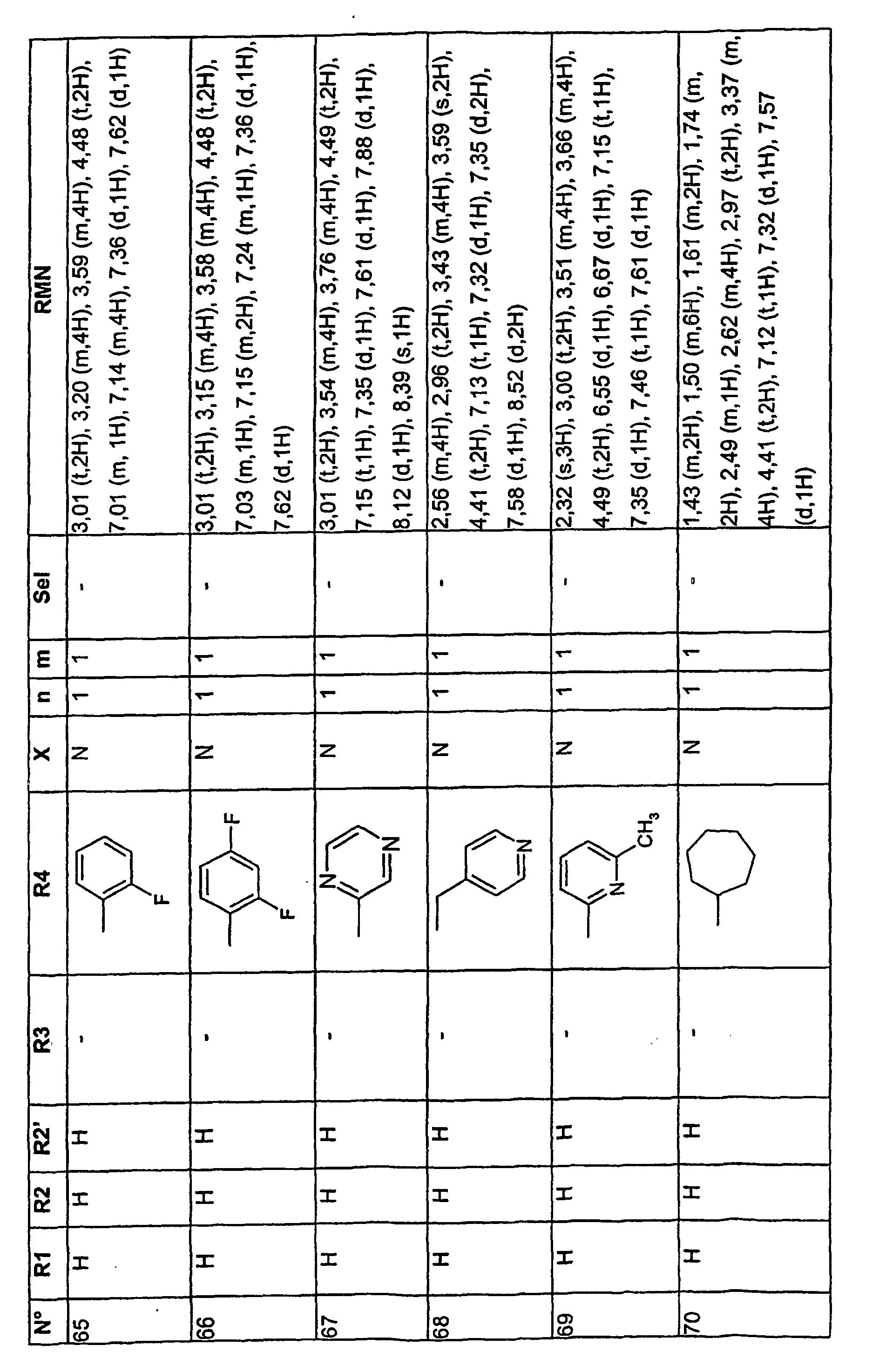

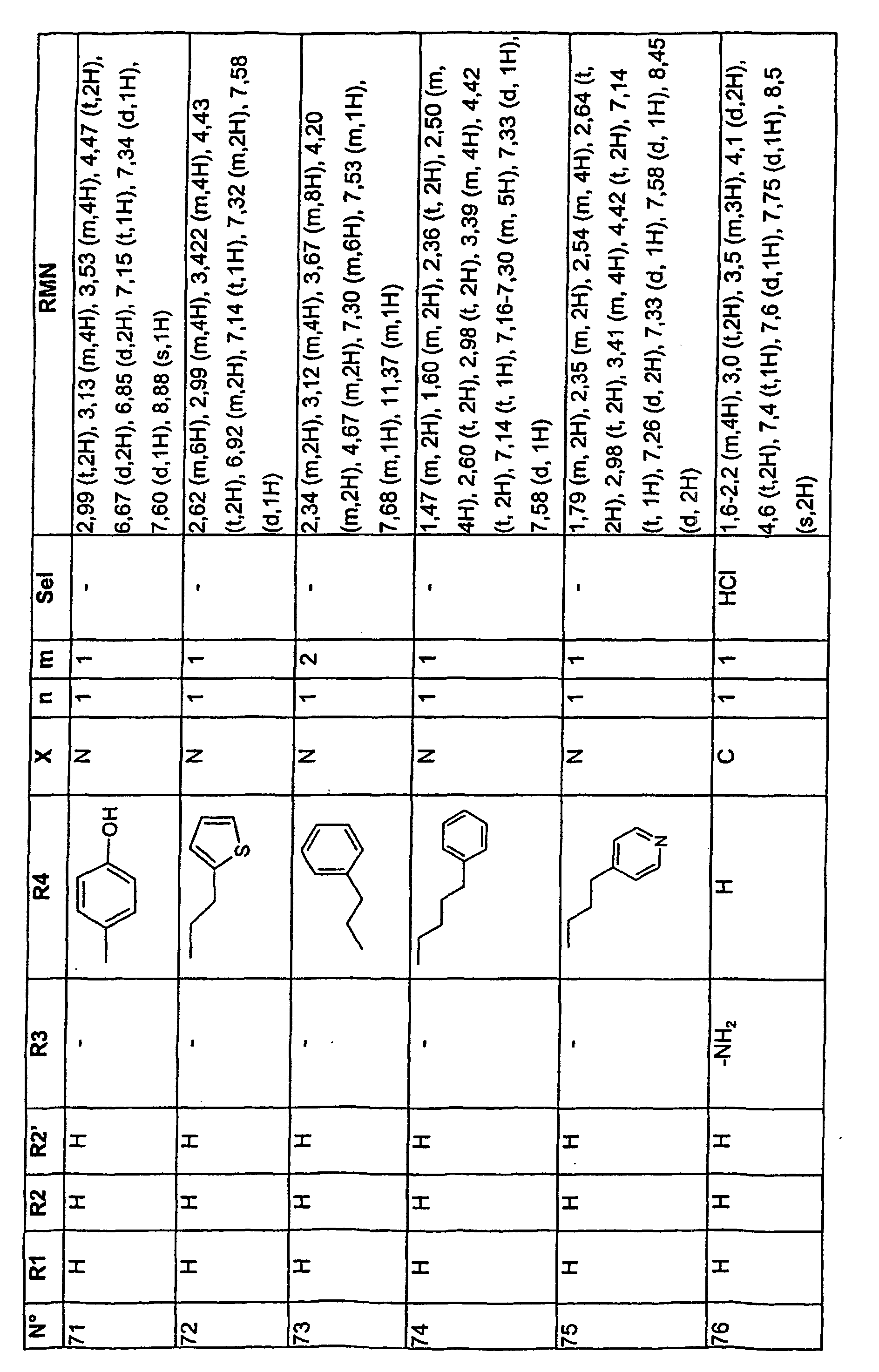

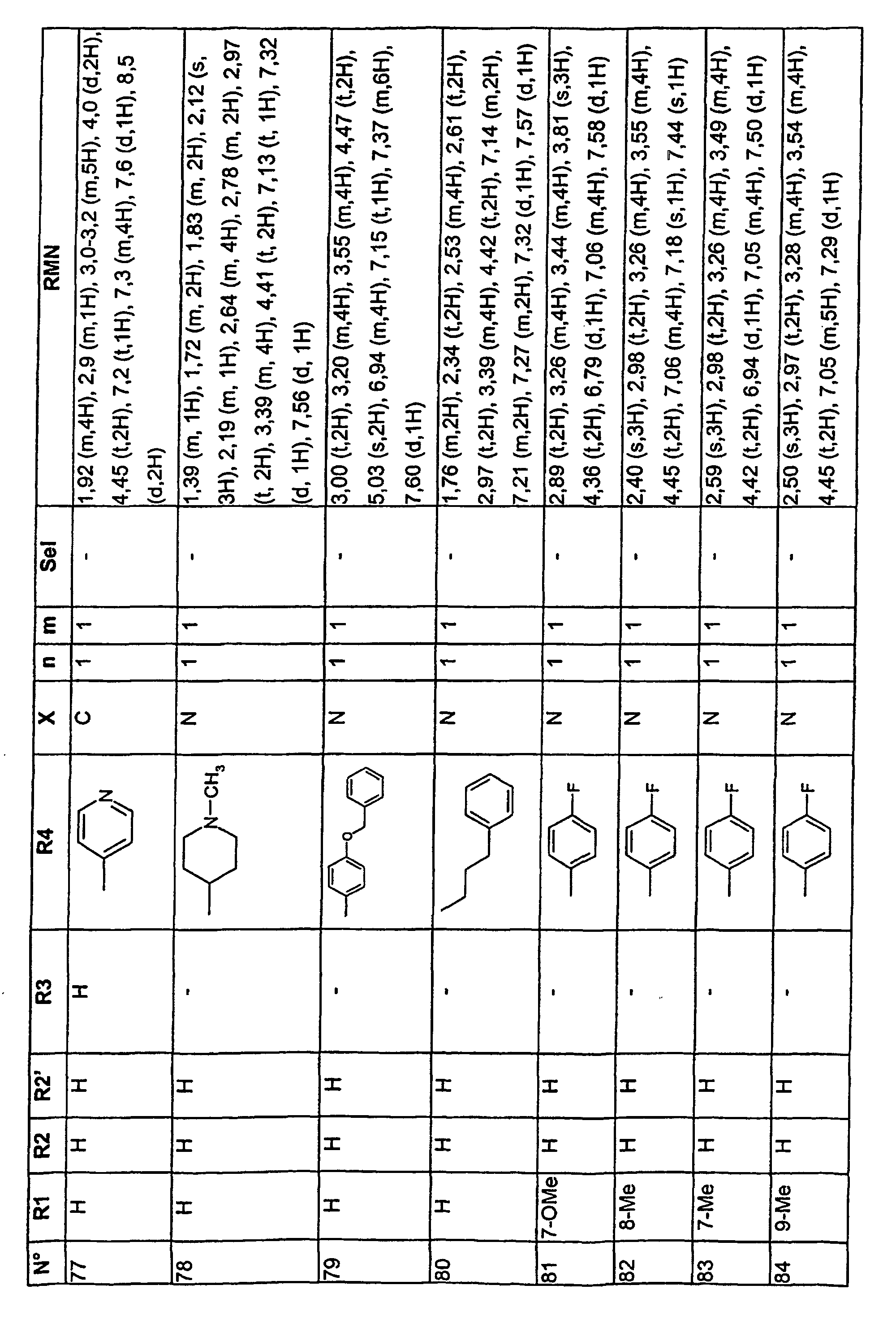

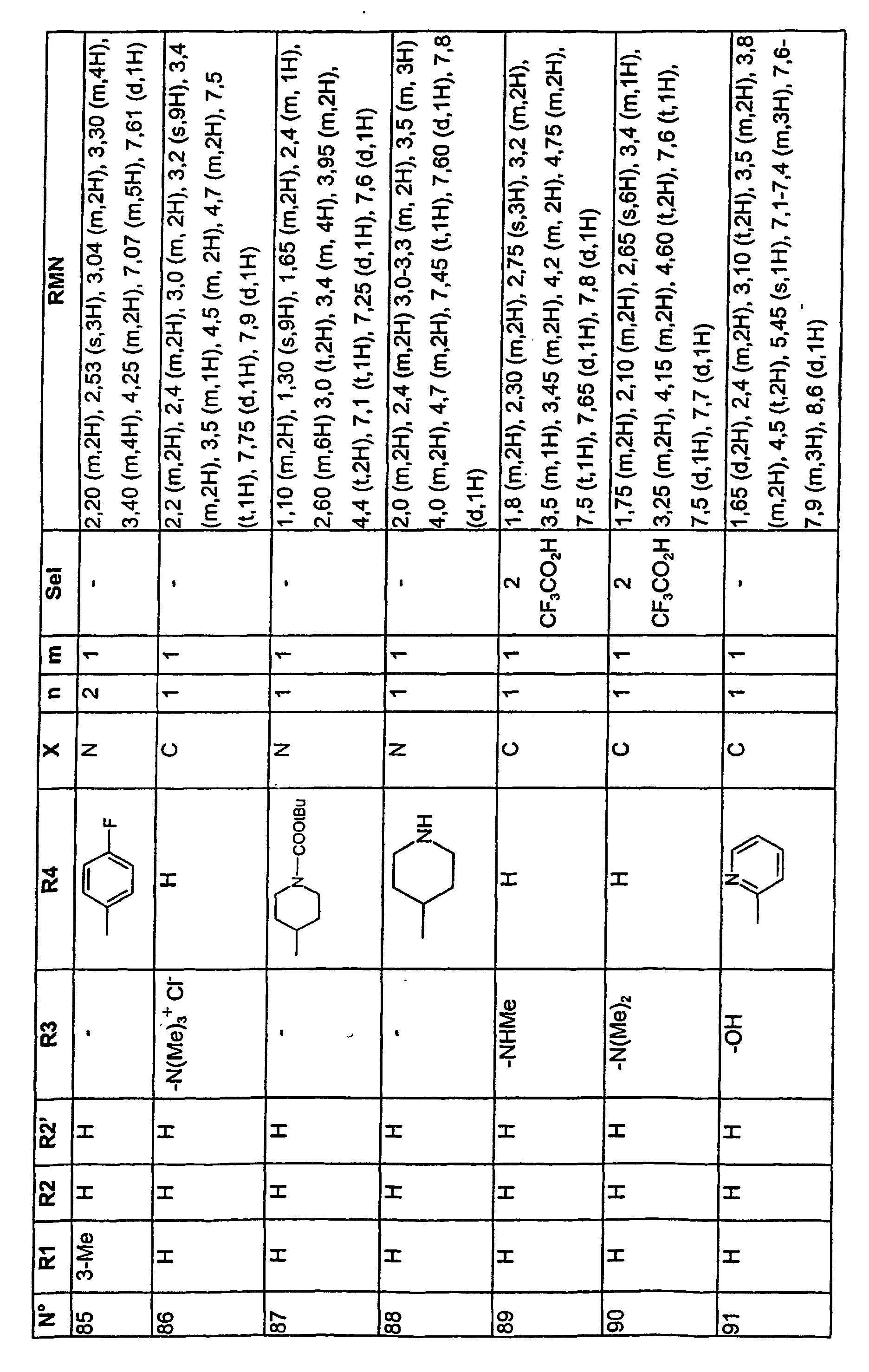

Les composés de l'invention ont fait l'objet d'essais pharmacologiques permettant de déterminer leur effet inhibiteur de la PARP ou poly(ADP-ribose)polymérase.

Les composés de l'invention ont été soumis au test suivant :

### Effets des composés sur l'activité enzymatique de la PARP

La PARP-1 humaine recombinante (hPARP-1) est produite par les cellules *Sf9* en utilisant un système d'expression baculovirus (Giner *et al.,* Gene (1992) **114** 279-283). L'enzyme est partiellement purifiée à partir de l'extrait cellulaire obtenu après précipitation par le sulfate d'ammonium à 70%. La solution de hPARP-1 obtenue est capable de générer 0,5-0,7 nmol de nicotinamide à partir du NAD+ dans les conditions d'essai standard décrites ci-dessous. Les composés à tester sont mis en solution dans un milieu d'incubation contenant 50 mM de tris-HCl, 10 mM de MgCl₂, 20 µM d'acétate de zinc, 1,5 mM de dithiothréitol, 0,2 µg d'histone et 0,1 µg d'oligonucléotide (GGAATTCC) pour 100 µl, en présence de hPARP-1 partiellement purifiée tamponnée à pH 8. La réaction enzymatique est engagée par l'addition de NAD+ (0,2 mM) et poursuivie à température ambiante pendant 20 minutes. La réaction est stoppée par l'addition de HClO₄ (1,2 M) à 4°C. Après centrifugation, les supemageants sont analysés en HPLC (colonne Shandon Ultrabase C8). L'élution isocratique est réalisée par un tampon phosphate (0,1 M) de pH 4,5 contenant 6% d'acétonitrile, injecté à 1,25 ml/min pendant 6 minutes. Le nicotinamide formé est détecté en mesurant l'absorbance UV de l'éluat à 265 nm et quantifié par rapport au pic formé par un standard externe de nicotinamide (2 nmoles). L'activité hPARP-1 résiduelle mesurée en présence de concentrations variables de composés de l'invention est comparée à celle obtenue en leur absence. Toutes les mesures sont faites au moins en duplicate et les Cl₅₀ sont calculées en utilisant l'équation de la sigmoïde de l'effet-dose.

Les composés les plus actifs dans cet essai se caractérisent par des Cl₅₀ comprises entre 5 et 500 nM.

Par ailleurs, les composés conformes à l'invention sont également actifs envers la PARP-2, les composés les plus actifs envers cette enzyme se caractérisant également par des Cl₅₀ comprises entre 5 et 500 nM.

Il apparaît donc que les composés de l'invention ont une activité inhibitrice sélective de la PARP, notamment de la PARP-1 et de la PARP-2.

Les composés de l'invention peuvent donc être utilisés pour la préparation de médicaments, en particulier de médicaments inhibiteurs de la PARP. Ces médicaments trouvent leur emploi en thérapeutique notamment dans la prévention ou le traitement de l'infarctus du myocarde, de l'ischémie cardiaque, l'insuffisance cardiaque, l'athérosclérose, la resténose après PTCA ou pontage, l'ischémie cérébrale et l'infarctus cérébral, causés par une ischémie, un traumatisme ou un accident thromboembolique, les maladies neurodégénératives comme la maladie de Parkinson, la maladie d'Alzheimer et la chorée de Huntington, l'insuffisance rénale aiguë, en particulier celle d'origine ischémique ou apparaissant après transplantation rénale, la transplantation cardiaque : traitement du rejet de greffe et de l'athérosclérose accélérée du greffon, les pathologies inflammatoires, les désordres immunologiques, les maladies rhumatoïdes, le diabète et les pancréatites, le choc septique, le syndrome de détresse respiratoire aiguë, les tumeurs et les métastases, les maladies autoimmunes, le SIDA, l'hépatite, le psoriasis, les vasculites, les colites ulcéreuses, les sclérose multiples et la myasthénie.

Ainsi, les composés de formule (I) conformes à l'invention peuvent être utilisés pour la préparation d'un médicament destiné au traitement et à la prévention des désordres dans lesquels l'enzyme PARP est impliquée.

Selon un autre aspect la présente invention a pour objet l'utilisation des composés de formule (I) pour lesquels R1 = R2 = R2' = R3 = H, X = C, n = m = 1 et R4 représente soit un groupe imidazol-4-yle, soit un groupe 5-méthyl-imidazol-4-yle, pour la fabrication d'un médicament destiné au traitement ou à la prévention des désordres dans lesquels l'enzyme poly(ADP-ribose)polymérase ou PARP est impliquée, tels que ceux mentionnés ci-dessus.

Enfin, la présente invention concerne également des compositions pharmaceutiques renfermant, en tant que principe actif, un composé selon l'invention. Ces compositions pharmaceutiques contiennent une dose efficace d'un composé selon l'invention, ainsi qu'un ou plusieurs excipients pharmaceutiques convenables. Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique ou rectale, le principe actif de formule (I) ci-dessus son sel ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

La dose de principe actif administrée par jour peut atteindre 0,1 à 1000 mg/kg par voie orale, parentérale ou rectale. Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Lorsqu'on prépare une composition solide sous forme de comprimés, on mélange l'ingrédient actif principal avec un excipient pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières. Les comprimés peuvent être réalisés par différentes techniques, telles que la compression directe, la granulation sèche, la granulation humide ou la fusion à chaud.

On obtient une préparation sous forme de gélules en mélangeant l'ingrédient actif avec un diluant et en versant le mélange obtenu dans des gélules molles ou dures.

Pour une administration parentérale, on peut utiliser des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

La présente invention selon un autre de ses aspects, concerne également une méthode de traitement des pathologies ci-dessus indiquées qui comprend l'administration d'un composé selon l'invention ou un de ses sels pharmaceutiquement acceptables.

## Revendications

1. Composés répondant à la formule (I) 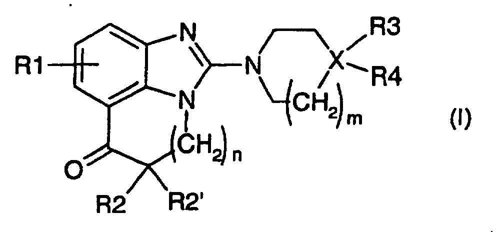 dans laquelle:
**R1** représente un atome d'hydrogène, un groupe (C1-C4)alkyle, un atome d'halogène, un groupe nitro ou un groupe (C1-C4)alcoxy,
**R2** et **R2'** représentent indépendamment l'un de l'autre un atome d'hydrogène ou un groupe (C1-C4)alkyle,
**X** représente un atome d'azote ou un atome de carbone,
**n** est égal à 1 ou 2,
**m** est égal à 1 ou 2,
**et dans le cas où X représente un atome d'azote :**
**R3** représente un atome d'hydrogène ou un groupe (C1-C4)alkyle pour donner des composés de formule (I) comportant un ammonium quaternaire ou encore n'existe pas pour donner des composés de formule (I) comportant une amine secondaire ou tertiaire,
**R4** représente
un atome d'hydrogène,
un groupe (C1-C6)alkyle,
un groupe (C3-C7)cycloalkyle,
un groupe (C3-C7)hétérocycloalkyle éventuellement substitué par un groupe (C1-C4)alkyle ou un groupe -COOR, où R représente un groupe (C1-C6)alkyle,
un groupe -(CH₂)ₚ-hétéroaryle, où p peut varier de 0 à 4 et où le groupe hétéroaryle est choisi parmi les groupes pyridyle, aminopyridyle, pyrimidinyle, pyrazinyle, pyridazinyle, imidazolyle et thiényle, ledit groupe hétéroaryle étant éventuellement substitué par un groupe (C1-C4)alkyle,
un groupe hétéroarylecarbonyle, le groupe hétéroaryle étant choisi parmi les groupes furyle, pyridyle, pyrimidyle, pyrazinyle, pyridazinyle et imidazolyle,
un groupe phénylcarbonyle, le groupe phényle étant éventuellement substitué par un atome d'halogène,
un groupe (C1-C6)alkylcarbonyle,
un groupe -(CH₂)ₚCOOR où p peut varier de 0 à 4 et où **R** représente un groupe (C1-C6)alkyle,
un groupe phénylsulfonyle éventuellement substitué sur le noyau phényle par un atome d'halogène, un groupe trifluorométhyle, un groupe (C1-C4)alkyle, un groupe nitro ou un groupe (C1-C4)alcoxy, ou bien
un groupe -(CH₂)ₚ phényle, où p peut varier de 0 à 4 et où le groupe phényle est éventuellement substitué par un à trois groupes choisis indépendamment l'un de l'autre parmi : un groupe (C1-C4)alkyle, un groupe nitro, un groupe amino, un groupe hydroxy, un atome d'halogène, un groupe trifluorométhyle, un groupe (C1-C4)alcoxy, un groupe (C1-C4)alcoxy-phényle, un groupe (C1-C4)alkylamino, un groupe (C1-C4)dialkylamino, un groupe -NHCHO ou un groupe -NHCOR', où R' représente un groupe (C1-C4)alcoxy ou un groupe (C1-C4)alkyle, ce groupe (C1-C4)alkyle pouvant être substitué par un groupe diméthylamino,
**et dans le cas où X représente un atome de carbone :**
**R3** représente un atome d'hydrogène, un groupe -NR5R6, un groupe -N(R5)₃⁺, un groupe -NHCOR7, un groupe -CONHR5, un groupe -COR7, un groupe -NHCONH₂, un groupe -OH ou un groupe -CH₂OH,
**R4** représente
un atome d'hydrogène,
un groupe -(CH₂)ₚ-phényle, où p peut varier de 0 à 4 et où le groupe phényle est éventuellement substitué par un à trois groupes choisis indépendamment l'un de l'autre parmi : un groupe (C1-C4)alkyle, un groupe nitro, un groupe amino, un atome d'halogène, un groupe trifluorométhyle ou un groupe (C1-C4)alcoxy,
un groupe -(CH₂)ₚ-hétéroaryle, où p peut varier de 0 à 4 et où le groupe hétéroaryle est choisi parmi un groupe pyridyle, un groupe,aminopyridyle, un groupe pyrimidinyle, un groupe pyrazinyle ou un groupe pyridazinyle, ou bien
un groupe -(CH₂)ₜNR7R8, où t est égal à 0 ou 1,
sous réserve que lorsque R4 représente un groupe -NR7R8, R3 est différent des groupes -NR5R6, -NHCOR7, -NHCONH₂, et -OH,
**R5** et **R6** représentent indépendamment l'un de l'autre un atome d'hydrogène ou un groupe (C1-C4)alkyle,
**R7** et **R8** représentent indépendamment l'un de l'autre un groupe (C1-C4)alkyle ou (C1-C4)alcoxy, ou forment ensemble un cycle saturé, comportant de 5 à 7 chaînons et comprenant éventuellement un atome d'azote supplémentaire, ce cycle pouvant être substitué, sur un atome de carbone ou bien un atome d'azote, y compris sur l'atome d'azote sur lequel sont liés les groupes R7 et R8 pour former un ammonium quaternaire, par un groupe (C1-C4)alkyle ou un groupe -COOR", où **R"** représente un groupe phényle ou (C1-C4)alkylphényle,
sous forme d'énantiomères, de diastéréoisomères ou de mélanges de ces différentes formes, y compris les mélanges racémiques, ainsi qu'à l'état de bases ou de sels d'addition à des acides pharmaceutiquement acceptables.

2. Composés de formule (I) selon la revendication 1, **caractérisés en ce que** :
**R1** représente un atome d'hydrogène, un groupe (C1-C4)alkyle ou un groupe (C1-C4)alcoxy,
**R2** représente un atome d'hydrogène ou un groupe (C1-C4)alkyle,
**R2'** représente un atome d'hydrogène,
**X** représente un atome d'azote,
**n** est égal à 1 ou 2,
**m** est égal à 1 ou 2,
**R3** représente un atome d'hydrogène pour donner des composés de formule (I) comportant un ammonium quaternaire ou encore n'existe pas pour donner des composés de formule (I) comportant une amine secondaire ou tertiaire,
**R4** représente
un atome d'hydrogène,
un groupe (C1-C6)alkyle,
un groupe (C3-C7)cycloalkyle,
un groupe pyridyle, pyrimidinyle ou pyrazinyle, éventuellement substitué par un groupe (C1-C4)alkyle,
un groupe hétéroarylecarbonyle, le groupe hétéroaryle étant choisi parmi un groupe furyle ou un groupe pyridyle,
un groupe phénylcarbonyle, le groupe phényle pouvant éventuellement être substitué par un atome d'halogène,
un groupe (C1-C6)alkylcarbonyle,
un groupe -(CH₂)ₚCOOR où p peut varier de 0 à 4 et où **R** représente un groupe (C1-C6)alkyle,
un groupe phénylsulfonyle,
un groupe phényle substitué par un à trois groupes choisis indépendamment l'un de l'autre parmi : un groupe (C1-C4)alkyle, un groupe nitro, un groupe amino, un groupe hydroxy, un atome d'halogène, un groupe trifluorométhyle, un groupe (C1-C4)alcoxy, un groupe (C1-C4)alcoxy-phényle, un groupe (C1-C4)dialkylamino, un groupe -NHCHO ou un groupe -NHCOR', où R' représente un groupe (C1-C4)alcoxy ou un groupe (C1-C4)alkyle, ce groupe (C1-C4)alkyle pouvant être substitué par un groupe diméthylamino,
un groupe -(CH₂)ₚ-phényle, où p peut varier de 0 à 4,
un groupe -(CH₂)ₚ-pyridyle, où p peut varier de 0 à 4,
un groupe -(CH₂)ₚ-thiényle, où p peut varier de 0 à 4,
un groupe (C3-C7)hétérocycloalkyle éventuellement substitué par un groupe (C1-C4)alkyle ou un groupe -COOR, où **R** représente un groupe (C1-C6)alkyle,
ou bien encore :
**R1** représente un atome d'hydrogène,
**R2** et **R2'** représentent indépendamment l'un de l'autre un atome d'hydrogène ou un groupe (C1-C4)alkyle,
**X** représente un atome de carbone,
**n** est égal à 1 ou 2,
**m** est égal à 1,
**R3** représente un atome d'hydrogène, un groupe -NR5R6, un groupe -N(R5)₃⁺, un groupe -NHCOR7, un groupe -CONHR5, un groupe -NHCONH₂, un groupe -OH ou un groupe -CH₂OH,
**R4** représente
un atome d'hydrogène,
un groupe benzyle,
un groupe phényle éventuellement substitué par un à trois groupes choisis indépendamment l'un de l'autre parmi: un groupe (C1-C4)alkyle, un groupe nitro, un groupe amino, un atome d'halogène, un groupe trifluorométhyle ou un groupe (C1-C4)alcoxy,
un groupe pyridyle,
un groupe -NR7R8,
sous réserve que lorsque R4 représente un groupe -NR7R8, R3 est différent des groupes -NR5R6, -NHCOR7, -NHCONH₂, et -OH,
**R5** et **R6** représentent indépendamment l'un de l'autre un atome d'hydrogène ou un groupe (C1-C4)alkyle,
**R7** et **R8** représentent indépendamment l'un de l'autre un groupe (C1-C4)alkyle ou (C1-C4)alcoxy, ou forment ensemble un cycle saturé, comportant de 5 à 7 chaînons et comprenant éventuellement un atome d'azote supplémentaire, ce cycle pouvant être substitué, sur un atome de carbone ou bien un atome d'azote, y compris sur l'atome d'azote sur lequel sont liés les groupes R7 et R8 pour former un ammonium quaternaire, par un groupe (C1-C4)alkyle ou un groupe -COOR", où **R"** représente un groupe phényle ou (C1-C4)alkylphényle,
sous forme d'énantiomères, de diastéréoisomères ou de mélanges de ces différentes formes, y compris les mélanges racémiques, ainsi qu'à l'état de bases ou de sels d'addition à des acides pharmaceutiquement acceptables.

3. Composés de formule (I) selon la revendication 1 ou la revendication 2, **caractérisés en ce que** :
**R1** représente un atome d'hydrogène, un groupe méthyle ou un groupe méthoxy,
**R2** représente un atome d'hydrogène ou un groupe méthyle,
**R2'** représente un atome d'hydrogène,
**X** représente un atome d'azote,
**n** est égal à 1 ou 2,
**m** est égal à 1 ou 2,
**R3** représente un atome d'hydrogène pour donner des composés de formule (I) comportant un ammonium quaternaire ou encore n'existe pas pour donner des composés de formule (I) comportant une amine secondaire ou tertiaire,
**R4** représente
un atome d'hydrogène,
un groupe (C1-C4)alkyle,
un groupe (C6-C7)cycloalkyle,
un groupe pyridyle, pyrimidinyle ou pyrazinyle, éventuellement substitué par un groupe (C1-C4)alkyle,
un groupe hétéroarylecarbonyle, le groupe hétéroaryle étant choisi parmi un groupe furyle ou un groupe pyridyle,
un groupe phénylcarbonyle, le groupe phényle pouvant éventuellement être substitué par un atome d'halogène,
un groupe (C3-C5)alkylcarbonyle,
un groupe -(CH₂)ₚCOOR où p est égal à 0 ou 1 et où **R** représente un groupe (C1-C4)alkyle,
un groupe phénylsulfonyle,
un groupe phényle substitué par un à trois groupes choisis indépendamment l'un de l'autre parmi : un groupe méthyle, un groupe nitro, un groupe amino, un groupe hydroxy, un atome d'halogène, un groupe trifluorométhyle, un groupe méthoxy, un groupe (C1-C4)alcoxy-phényle, un groupe diméthylamino, un groupe -NHCHO ou un groupe -NHCOR', où **R'** représente un groupe (C1-C4)alcoxy ou un groupe (C1-C4)alkyle, ce groupe (C1-C4)alkyle pouvant être substitué par un groupe diméthylamino,
un groupe -(CH₂)ₚ-phényle, où p est égal à 1, 2, 3 ou 4,
un groupe -(CH₂)ₚ-pyridyle, où p peut varier de 1 à 3,
un groupe -(CH₂)ₚ-thiényle, où p est égal à 2,
un groupe (C6-C7)hétérocycloalkyle éventuellement substitué par un groupe méthyle ou un groupe -COOR, où **R** représente un groupe (C1-C4)alkyle,
ou bien encore :
**R1** représente un atome d'hydrogène,
**R2** et **R2'** représentent indépendamment l'un de l'autre un atome d'hydrogène ou un groupe méthyle,
**X** représente un atome de carbone,
**n** est égal à 1 ou 2,
**m** est égal à 1,
**R3** représente un atome d'hydrogène, un groupe -NR5R6, un groupe -N(CH₃)₃⁺, un groupe -NHCOR7, un groupe -CONHR5, un groupe -NHCONH₂, un groupe -OH ou un groupe -CH₂OH,
**R4** représente
un atome d'hydrogène,
un groupe benzyle,
un groupe phényle éventuellement substitué par un à trois groupes choisis indépendamment l'un de l'autre parmi un atome d'halogène ou un groupe trifluorométhyle,
un groupe pyridyle,
un groupe -NR7R8,
sous réserve que lorsque R4 représente un groupe -NR7R8, R3 est différent des groupes -NR5R6, -NHCOR7, -NHCONH₂, et -OH,
**R5** et **R6** représentent indépendamment l'un de l'autre un atome d'hydrogène ou un groupe (C1-C4)alkyle,
**R7** et **R8** représentent indépendamment l'un de l'autre un groupe (C1-C4)alkyle ou forment ensemble un cycle saturé, comportant de 5 à 7 chaînons et comprenant éventuellement un atome d'azote supplémentaire, ce cycle pouvant être substitué, sur un atome de carbone ou bien un atome d'azote, y compris sur l'atome d'azote sur lequel sont liés les groupes R7 et R8 pour former un ammonium quaternaire, par un groupe méthyle ou un groupe -COOR", où **R"** représente un groupe (C1-C4)alkylphényle,
sous forme d'énantiomères, de diastéréoisomères ou de mélanges de ces différentes formes, y compris les mélanges racémiques, ainsi qu'à l'état de bases ou de sels d'addition à des acides pharmaceutiquement acceptables.

4. Composés selon l'une quelconque des revendications 1 à 3, **caractérisés en ce qu'**ils sont choisis parmi :
• 1-(6-oxo-5,6-dihydro-4*H*-imidazo[4,5,1-ij]quinoléin-2-yl)-4-pipéridinopipéridine
• 1-méthyl-1-[1-(6-oxo-5,6-dihydro-4*H*-imidazo[4,5,1-*ij*]quinoléin-2-yl)-4-pipéridinyl]-pipéridine
• 2-[4-(4-diméthylaminophényl)pipérazin-1-yl]4,5-dihydro-imidazo-[4,5,1-ij]quinoléin-6-one
• 2-[4-(4-*tert*-butoxycarbonylaminophényl)pipérazin-1-yl]-4,5-dihydro-imidazo[4,5,1-ij]quinoléin-6-one
• 2-[4-(4-aminophényl)pipérazin-1-yl]4,5-dihydro-imidazo[4,5,1-ij]quinoléin-6-one
• 2-[4-(4-acétamidophény)pipérazin-1yl]4,5-dihydro-imidazo [4,5,1-ij]quinoléin-6-one
• 2-(4-n-propyl-pipérazin-1-yl)-4,5-dihydro-imidazo[4,5,1-ij]quinoléin-6-one
• 2-(4-*tert*-butyloxycarbonyl-pipérazin-1-yl)-4,5-dihydro-imidazo[4,5,1-ij]quinoléin-6-one
• 2-(pipérazin-1-yl)-4,5-dihydro-imidazo[4,5,1-ij]quinoléin-6-one
• 2-(4-acétyl-pipérazin-1-yl)-4,5-dihydro-imidazo[4,5,1-ij]quinoléin-6-one
• 2-[4-(4-pyridyl)-pipérazin-1-yl)-4,5-dihydro-imidazo[4,5,1-ij]quinoléin-6-one
• 2-(4-benzyl-pipéridin-1-yl)-4,5-dihydro-imidazo[4,5,1-ij]quinoléin-6-one
• 1-(6-oxo-5,6-dihydro-4*H*-imidazo[4,5,1-ij]quinoléin-2-yl)-4-phényl-4-pipéridinecarboxamide
• 1-(6-oxo-5,6-d)hydro-4*H*-imidazo[4,5,1-ij]quinoléin-2-yl)-4-phényl-4-aminopipéridine
• 2-(4-(4-hydroxyphényl)-pipérazin-1-yl]-4,5-dihydro-imidazo [4,5,1-ij]quinoléin-6-one
• 2-[4-(4-fluorophényl)-pipérazin-1-yl]-4,5-dihydro-9-méthyl-imidazo[4,5,1-ij]quinoléin-6-one
• 1-[4-(4-fluorophényl)-pipérazin-1-yl]-3-méthyl-8,9-dihydro-7*H*-2,9a-diazabenzo[cd]azulen-6-one
• 2-[4-(4-fluorophényl)-pipérazin-1-yl]-4,5-dihydro-7-méthyl-imidazo[4,5,1-ij]quinoléin-6-one
• 2-[4-(4-fluorophényl)-pipérazin-1-yl]-4,5-dihydro-8-méthyl-imidazo[4,5,1-ij]quinoléin-6-one
• 2-[4-(4-fluorophényl)-pipérazin-1-yl]-4,5-dihydro-7-méthoxy-imidazo[4,5,1-ij]quinoléin-6-one
• 2-[4-(4-méthoxyphényl)-pipérazin-1-yl]-4,5-dihydro-imidazo [4,5,1-ij]quinoléin-6-one
• 1-(6-oxo-5,6-dihydro-4*H*-imidazo[4,5,1-ij]quinoléin-2-yl)-4*H*-4-diméthylaminopipéridine
sous forme d'énantiomères, de diastéréoisomères ou de mélanges de ces différentes formes, y compris les mélanges racémiques, ainsi qu'à l'état de bases ou de sels d'addition à des acides pharmaceutiquement acceptables.

5. Procédé de préparation d'un composé de formule (I) selon l'une quelconque des revendications 1 à 4, **caractérisé en ce que** l'on fait réagir un dérivé de formule (II) : 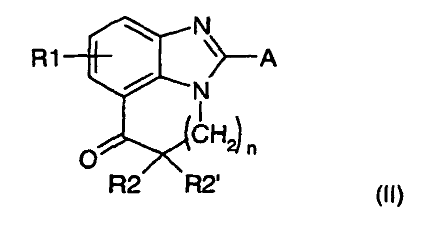 dans laquelle R1, R2, R2' et n sont tels que définis dans la revendication 1 et A représente un groupe partant, en présence d'une amine de formule (III) : 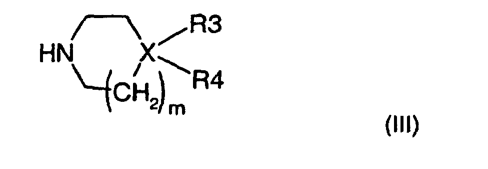 dans laquelle X, R3, R4 et m sont tels que définis dans la revendication 1, dans un solvant qui peut être un alcool, un éther ou bien un hydrocarbure pour obtenir un composé de formule (I) selon la revendication 1, la réaction pouvant s'effectuer en présence d'une base, en présence d'halogénures de métal alcalin ou en présence de catalyseurs à base de palladium ou de nickel.

6. Composés de formule (II) telle que définie dans la revendication 5, utiles en tant qu'intermédiaires de synthèse.

7. Médicament, **caractérisé en ce qu'**il comprend un composé de formule (I) selon l'une quelconque des revendications 1 à 4.

8. Composition pharmaceutique, **caractérisée en ce qu'**elle comprend un composé de formule (I) selon l'une quelconque des revendications 1 à 4, ainsi qu'un ou plusieurs excipients pharmaceutiques convenables.

9. Utilisation d'un composé de formule (I) selon l'une quelconque des revendications 1 à 4 pour la préparation d'un médicament destiné à la prévention ou au traitement de l'infarctus du myocarde, de l'ischémie cardiaque, l'insuffisance cardiaque, l'athérosclérose, la resténose après PTCA ou pontage, l'ischémie cérébrale et l'infarctus cérébral, causés par une ischémie, un traumatisme ou un accident thromboembolique, les maladies neurodégénératives comme la maladie de Parkinson, la maladie d'Alzheimer et la chorée de Huntington, l'insuffisance rénale aiguë, en particulier celle d'origine ischémique ou apparaissant après transplantation rénale, la transplantation cardiaque : traitement du rejet de greffe et de l'athérosclérose accélérée du greffon, les pathologies inflammatoires, les désordres immunologiques, les maladies rhumatoïdes, le diabète et les pancréatites, le choc septique, le syndrome de détresse respiratoire aiguë, les tumeurs et les métastases, les maladies autoimmunes, le SIDA, l'hépatite, le psoriasis, les vasculites, les colites ulcéreuses, les sclérose multiples et la myasthénie.

10. Utilisation des composés de formule (I) selon la revendication 1, dans lesquels R1 = R2 = R2' = R3 = H, X = C, n = m = 1 et R4 représente soit un groupe imidazol-4-yle, soit un groupe 5-méthyl-imidazol-4-yle, pour la préparation d'un médicament destiné à la prévention ou au traitement de l'infarctus du myocarde, de l'ischémie cardiaque, l'insuffisance cardiaque, l'athérosclérose, la resténose après PTCA ou pontage, l'ischémie cérébrale et l'infarctus cérébral, causés par une ischémie, un traumatisme ou un accident thromboembolique, la maladie de Parkinson, la chorée de Huntington, l'insuffisance rénale aiguë, le rejet de greffe et l'athérosclérose accélérée du greffon, les pathologies inflammatoires, les désordres immunologiques, les maladies rhumatoïdes, le diabète et les pancréatites, le choc septique, le syndrome de détresse respiratoire aiguë, les tumeurs et les métastases, les maladies autoimmunes, le SIDA, l'hépatite, le psoriasis, les vasculites, les colites ulcéreuses, les sclérose multiples et la myasthénie.

11. Utilisation des composés de formule (I) selon la revendication 1, dans lesquels X représente un atome de carbone, n, m, R1, R2, R2' et R3 sont tels que définis dans l'une quelconque des revendications 1 à 4, et R4 représente un groupe -(CH₂)ₚ-hétéroaryle, où p peut varier de 0 à 4 et où le groupe hétéroaryle est un groupe imidazolyle éventuellement substitué par un groupe (C1-C4)alkyle, pour la préparation d'un médicament destiné à la prévention ou au traitement de l'infarctus du myocarde, de l'ischémie cardiaque, l'insuffisance cardiaque, l'athérosclérose, la resténose après PTCA ou pontage, l'ischémie cérébrale et l'infarctus cérébral, causés par une ischémie, un traumatisme ou un accident thromboembolique, la maladie de Parkinson, la chorée de Huntington, l'insuffisance rénale aiguë, le rejet de greffe et l'athérosclérose accélérée du greffon, les pathologies inflammatoires, les désordres immunologiques, les maladies rhumatoïdes, le diabète et les pancréatites, le choc septique, le syndrome de détresse respiratoire aiguë, les tumeurs et les métastases, les maladies autoimmunes, le SIDA, l'hépatite, le psoriasis, les vasculites, les colites ulcéreuses, les sclérose multiples et la myasthénie.

## Claims

1. Compounds corresponding to formula (I) 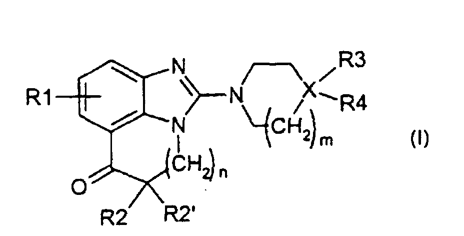 in which:
**R1** represents a hydrogen atom, a C1-C4 alkyl group, a halogen atom, a nitro group or a C1-C4 alkoxy group,
**R2** and **R2'** represent, independently of each other, a hydrogen atom or a C1-C4 alkyl group,
**X** represents a nitrogen atom or a carbon atom,
**n** is equal to 1 or 2,
**m** is equal to 1 or 2,
**and, when X represents a nitrogen atom:**
**R3** represents a hydrogen atom or a C1-C4 alkyl group, to give compounds of formula (I) comprising a quaternary ammonium, or alternatively does not exist, to give compounds of formula (I) comprising a secondary or tertiary amine,
**R4** represents
a hydrogen atom,
a C1-C6 alkyl group,
a C3-C7 cycloalkyl group,
a C3-C7 heterocycloalkyl group optionally substituted with a C1-C4 alkyl group or a group -COOR, in which **R** represents a C1-C6 alkyl group,
a group -(CH₂)ₚ-heteroaryl, in which p may range from 0 to 4 and in which the heteroaryl group is chosen from pyridyl, aminopyridyl, pyrimidinyl, pyrazinyl, pyridazinyl, imidazolyl and thienyl groups, the said heteroaryl group optionally being substituted with a C1-C4 alkyl group,
a heteroarylcarbonyl group, the heteroaryl group being chosen from furyl, pyridyl, pyrimidyl, pyrazinyl, pyridazinyl and imidazolyl groups,
a phenylcarbonyl group, the phenyl group optionally being substituted with a halogen atom,
a (C1-C6)alkylcarbonyl group,
a group -(CH₂)ₚCOOR in which p may range from 0 to 4 and in which R represents a C1-C6 alkyl group,
a phenylsulphonyl group optionally substituted on the phenyl nucleus with a halogen atom, a trifluoromethyl group, a C1-C4 alkyl group, a nitro group or a C1-C4 alkoxy group, or alternatively
a -(CH₂)ₚ-phenyl group, in which p may range from 0 to 4 and in which the phenyl group is optionally substituted with one to three groups chosen, independently of each other, from: a C1-C4 alkyl group, a nitro group, an amino group, a hydroxyl group, a halogen atom, a trifluoromethyl group, a C1-C4 alkoxy group, a (C1-C4)alkoxyphenyl group, a C1-C4 alkylamino group, a C1-C4 dialkylamino group, an -NHCHO group or a group -NHCOR', in which **R'** represents a C1-C4 alkoxy group or a C1-C4 alkyl group, this C1-C4 alkyl group optionally being substituted with a dimethylamino group,
**and, when X represents a carbon atom:**
**R3** represents a hydrogen atom, a group -NR5R6, a group -N(R5)₃⁺, a group -NHCOR7, a group -CONHR5, a group -COR7, an -NHCONH₂ group, an -OH group or a -CH₂OH group,
**R4** represents
a hydrogen atom,
a -(CH₂)ₚ-phenyl group, in which p may range from 0 to 4 and in which the phenyl group is optionally substituted with one to three groups chosen, independently of each other, from: a C1-C4 alkyl group, a nitro group, an amino group, a halogen atom, a trifluoromethyl group or a C1-C4 alkoxy group,
a -(CH₂)ₚ-heteroaryl group, in which p may range from 0 to 4 and in which the heteroaryl group is chosen from a pyridyl group, an aminopyridyl group, a pyrimidinyl group, a pyrazinyl group or a pyridazinyl group, or alternatively
a group -(CH₂)ₜNR7R8, in which t is equal to 0 or 1,
with the proviso that when R4 represents a group -NR7R8, R3 is other than the groups -NR5R6, -NHCOR7, -NHCONH₂ and -OH,
**R5** and **R6** represent, independently of each other, a hydrogen atom or a C1-C4 alkyl group,
**R7** and **R8** represent, independently of each other, a C1-C4 alkyl or C1-C4 alkoxy group or together form a saturated 5- to 7-membered ring optionally comprising an additional nitrogen atom, this ring optionally being substituted, on a carbon atom or on a nitrogen atom, including the nitrogen atom to which the groups R7 and R8 are attached to form a quaternary ammonium, with a C1-C4 alkyl group or a group -COOR", in which **R"** represents a phenyl or (C1-C4)alkylphenyl group,
in the form of enantiomers or diastereoisomers or mixtures of these various forms, including racemic mixtures, and also in the form of bases or addition salts with pharmaceutically acceptable acids.

2. Compounds of formula (I) according to Claim 1, **characterized in that**:
**R1** represents a hydrogen atom, a C1-C4 alkyl group or a C1-C4 alkoxy group,
**R2** represents a hydrogen atom or a C1-C4 alkyl group,
**R2'** represents a hydrogen atom,
**X** represents a nitrogen atom,
**n** is equal to 1 or 2,
**m** is equal to 1 or 2,
**R3** represents a hydrogen atom, to give compounds of formula (I) comprising a quaternary ammonium, or alternatively does not exist, to give compounds of formula (I) comprising a secondary or tertiary amine,
**R4** represents
a hydrogen atom,
a C1-C6 alkyl group,
a C3-C7 cycloalkyl group,
a pyridyl, pyrimidinyl or pyrazinyl group, optionally substituted with a C1-C4 alkyl group,
a heteroarylcarbonyl group, the heteroaryl group being chosen from a furyl group and a pyridyl group,
a phenylcarbonyl group, the phenyl group optionally being substituted with a halogen atom,
a (C1-C6)alkylcarbonyl group,
a group -(CH₂)ₚCOOR in which p can range from 0 to 4 and in which **R** represents a C1-C6 alkyl group,
a phenylsulphonyl group,
a phenyl group substituted with one to three groups chosen, independently of each other, from: a C1-C4 alkyl group, a nitro group, an amino group, a hydroxyl group, a halogen atom, a trifluoromethyl group, a C1-C4 alkoxy group, a (Cl-C4)alkoxyphenyl group, a (C1-C4)-dialkylamino group, an -NHCHO group or a group -NHCOR', in which **R'** represents a C1-C4 alkoxy group or a C1-C4 alkyl group, this C1-C4 alkyl group optionally being substituted with a dimethylamino group,
a -(CH₂)ₚ-phenyl group, in which p can range from 0 to 4,
a -(CH₂)ₚ-pyridyl group, in which p can range from 0 to 4,
a -(CH₂)ₚ-thienyl group, in which p can range from 0 to 4,
a (C3-C7)heterocycloalkyl group optionally substituted with a C1-C4 alkyl group or a group -COOR, in which **R** represents a C1-C6 alkyl group,
or alternatively:
**R1** represents a hydrogen atom,
**R2** and **R2'** represent, independently of each other, a hydrogen atom or a C1-C4 alkyl group,
**X** represents a carbon atom,
**n** is equal to 1 or 2,
**m** is equal to 1,
**R3** represents a hydrogen atom, a group -NR5R6, a group -N(R5)₃⁺, a group -NHCOR7, a group -CONHR5, an -NHCONH₂ group, an -OH group or a -CH₂OH group,
**R4** represents
a hydrogen atom,
a benzyl group,
a phenyl group optionally substituted with one to three groups chosen, independently of each other, from: a C1-C4 alkyl group, a nitro group, an amino group, a halogen atom, a trifluoromethyl group or a C1-C4 alkoxy group,
a pyridyl group,
a group -NR7R8,
with the proviso that when R4 represents a group -NR7R8, R3 is other than the groups -NR5R6, -NHCOR7, -NHCONH₂ and -OH,
**R5** and **R6** represent, independently of each other, a hydrogen atom or a C1-C4 alkyl group,
**R7** and **R8** represent, independently of each other, a C1-C4 alkyl or C1-C4 alkoxy group, or together form a saturated 5- to 7-membered ring optionally comprising an additional nitrogen atom, this ring optionally being substituted, on a carbon atom or a nitrogen atom, including the nitrogen atom to which the groups R7 and R8 are attached to form a quaternary ammonium, with a C1-C4 alkyl group or a group -COOR", in which **R"** represents a phenyl or (C1-C4)alkylphenyl group,
in the form of enantiomers or diastereoisomers or mixtures of these various forms, including racemic mixtures, and also in the form of bases or addition salts with pharmaceutically acceptable acids.

3. Compounds of formula (I) according to Claim 1 or Claim 2, **characterized in that**:
**R1** represents a hydrogen atom, a methyl group or a methoxy group,
**R2** represents a hydrogen atom or a methyl group,
**R2'** represents a hydrogen atom,
**X** represents a nitrogen atom,
**n** is equal to 1 or 2,
**m** is equal to 1 or 2,
**R3** represents a hydrogen atom, to give compounds of formula (I) comprising a quaternary ammonium, or alternatively does not exist, to give compounds of formula (I) comprising a secondary or tertiary amine,
**R4** represents
a hydrogen atom,
a C1-C4 alkyl group,
a C6-C7 cycloalkyl group,
a pyridyl, pyrimidinyl or pyrazinyl group, optionally substituted with a C1-C4 alkyl group,
a heteroarylcarbonyl group, the heteroaryl group being chosen from a furyl group and a pyridyl group,
a phenylcarbonyl group, the phenyl group optionally being substituted with a halogen atom,
a (C3-C5)alkylcarbonyl group,
a group -(CH₂)ₚCOOR in which p is equal to 0 or 1 and in which **R** represents a C1-C4 alkyl group,
a phenylsulphonyl group,
a phenyl group substituted with one to three groups chosen, independently of each other, from: a methyl group, a nitro group, an amino group, a hydroxyl group, a halogen atom, a trifluoromethyl group, a methoxy group, a (C1-C4)alkoxyphenyl group, a dimethylamino group, an -NHCHO group or a group -NHCOR', in which **R'** represents a C1-C4 alkoxy group or a C1-C4 alkyl group, this C1-C4 alkyl group optionally being substituted with a dimethylamino group,
a -(CH₂)ₚ-phenyl group, in which p is equal to 1, 2, 3 or 4,
a -(CH₂)ₚ-pyridyl group, in which p can range from 1 to 3,
a -(CH₂)ₚ-thienyl group, in which p is equal to 2,
a C6-C7 heterocycloalkyl group optionally substituted with a methyl group or a group -COOR, in which **R** represents a C1-C4 alkyl group,
or alternatively:
**R1** represents a hydrogen atom,
**R2** and **R2'** represent, independently of each other, a hydrogen atom or a methyl group,
**X** represents a carbon atom,
**n** is equal to 1 or 2,
**m** is equal to 1,
**R3** represents a hydrogen atom, a group -NR5R6, an -N(CH₃)₃⁺ group, a group -NHCOR7, a group -CONHR5, an -NHCONH₂ group, an -OH group or a -CH₂OH group,
**R4** represents
a hydrogen atom,
a benzyl group,
a phenyl group optionally substituted with one to three groups chosen, independently of each other, from a halogen atom and a trifluoromethyl group,
a pyridyl group,
a group -NR7R8,
with the proviso that when R4 represents a group -NR7R8, R3 is other than the groups -NR5R6, -NHCOR7, -NHCONH₂ and -OH,
**R5** and **R6** represent, independently of each other, a hydrogen atom or a C1-C4 alkyl group,
**R7** and **R8** represent, independently of each other, a C1-C4 alkyl group or together form a saturated 5- to 7-membered ring optionally comprising an additional nitrogen atom, this ring optionally being substituted, on a carbon atom or on a nitrogen atom, including the nitrogen atom to which the groups R7 and R8 are attached to form a quaternary ammonium, with a methyl group or a group -COOR", in which **R"** represents a (C1-C4)alkylphenyl group,
in the form of enantiomers or diastereoisomers or mixtures of these various forms, including racemic mixtures, and also in the form of bases or addition salts with pharmaceutically acceptable acids.

4. Compounds according to any one of Claims 1 to 3, **characterized in that** they are chosen from:
• 1-(6-oxo-5,6-dihydro-4*H*-imidazo[4,5,1-ij]quinolin-2-yl)-4-piperidinopiperidine
• 1-methyl-1-[1-(6-oxo-5,6-dihydro-4*H*-imidazo[4,5,1-*ij*]quinolin-2-yl)-4-piperidinyl]piperidine
• 2-[4-(4-dimethylaminophenyl)piperazin-1-yl]-4,5-dihydroimidazo-[4,5,1-ij]quinolin-6-one
• 2-[4-(4-*tert*-butoxycarbonylaminophenyl)piperazin-1-yl]-4,5-dihydroimidazo[4,5,1-ij]quinolin-6-one
• 2-[4-(4-aminophenyl)piperazin-1-yl]4,5-dihydroimidazo[4,5,1-ij]quinolin-6-one
• 2-[4-(4-acetamidophenyl)piperazin-1-yl]-4,5-dihydroimidazo[4,5,1-ij]quinolin-6-one
• 2-(4-n-propylpiperazin-1-yl)-4,5-dihydroimidazo[4,5,1-ij]quinolin-6-one
• 2-(4-*tert*-butyloxycarbonylpiperazin-1-yl)-4,5-dihydroimidazo[4,5,1-ij]quinolin-6-one
• 2-(piperazin-1-yl)-4,5-dihydroimidazo[4,5,1-ij]quinolin-6-one
• 2-(4-acetylpiperazin-1-yl)-4,5-dihydroimidazo[4,5,1-ij]quinolin-6-one
• 2-[4-(4-pyridyl)piperazin-1-yl)-4,5-dihydroimidazo[4,5,1-ij]quinolin-6-one
• 2-(4-benzylpiperidin-1-yl)-4,5-dihydroimidazo[4,5,1-ij]quinolin-6-one
• 1-(6-oxo-5,6-dihydro-4*H*-imidazo[4,5,1-ij]quinolin-2-yl)-4-phenyl-4-piperidinecarboxamide
• 1-(6-oxo-5,6-dihydro-4*H*-imidazo[4,5,1-ij]quinolin-2-yl)-4-phenyl-4-aminopiperidine
• 2-[4-(4-hydroxyphenyl)piperazin-1-yl]-4,5-dihydroimidazo[4,5,1-ij]quinolin-6-one
• 2-[4-(4-fluorophenyl)piperazin-1-yl]-4,5-dihydro-9-methylimidazo[4,5,1-ij]quinolin-6-one
• 1-[4-(4-fluorophenyl)piperazin-1-yl]-3-methyl-8,9-dihydro-7*H*-2,9a-diazabenzo[cd]azulen-6-one
• 2-[4-(4-fluorophenyl)piperazin-1-yl]-4,5-dihydro-7-methylimidazo[4,5,1-ij]quinolin-6-one
• 2-[4-(4-fluorophenyl)piperazin-1-yl]-4,5-dihydro-8-methylimidazo[4,5,1-ij]quinolin-6-one
• 2-[4-(4-fluorophenyl)piperazin-1-yl]-4,5-dihydro-7-methoxyimidazo[4,5,1-ij]quinolin-6-one
• 2-[4-(4-methoxyphenyl)piperazin-1-yl]-4,5-dihydroimidazo[4,5,1-ij]quinolin-6-one
• 1-(6-oxo-5,6-dihydro-4*H*-imidazo[4,5,1-ij]quinolin-2-yl)-4*H*-4-dimethylaminopiperidine
in the form of enantiomers or diastereoisomers or mixtures of these various forms, including racemic mixtures, and also in the form of bases or addition salts with pharmaceutically acceptable acids.

5. Process for preparing a compound of formula (I) according to any one of Claims 1 to 4,
**characterized in that** a derivative of formula (II) : 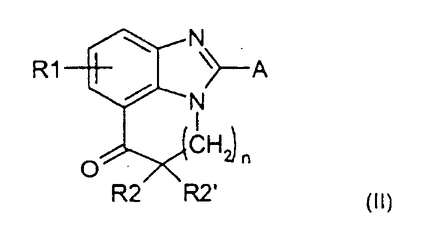 in which R1, R2, R2' and n are as defined in Claim 1 and A represents a leaving group, is reacted in the presence of an amine of formula (III): 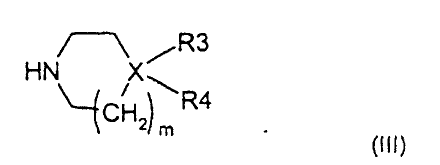 in which X, R3, R4 and m are as defined in Claim 1, in a solvent which may be an alcohol, an ether or a hydrocarbon, to give a compound of formula (I) according to Claim 1, the reaction being able to be carried out in the presence of a base, in the presence of alkali metal halides or in the presence of palladium-based or nickel-based catalysts.

6. Compounds of formula (II) as defined in Claim 5, which are useful as synthetic intermediates.

7. Medicinal product, **characterized in that** it comprises a compound of formula (I) according to any one of Claims 1 to 4.

8. Pharmaceutical composition, **characterized in that** it comprises a compound of formula (I) according to any one of Claims 1 to 4, and also one or more suitable pharmaceutical excipients.

9. Use of a compound of formula (I) according to any one of Claims 1 to 4, for the preparation of a medicinal product for preventing or treating myocardial infarction, cardiac ischaemia, cardiac insufficiency, atherosclerosis, restenosis after PTCA or bypass, cerebral ischaemia and cerebral infarction, caused by an ischaemia, a trauma or a thromboembolic accident, neurodegenerative diseases such as Parkinson's disease, Alzheimer's disease and Huntington's chorea, acute renal insufficiency, in particular that of ischaemic origin or appearing after kidney transplant, heart transplant: treatment of graft rejection and accelerated atherosclerosis of grafts, inflammatory pathologies, immunological disorders, rheumatoid diseases, diabetes and pancreatitis, septic shock, acute respiratory distress syndrome, tumours and metastases, autoimmune diseases, AIDS, hepatitis, psoriasis, vasculitis, ulcerative colitis, multiple sclerosis and myasthenia.

10. Use of the compounds of formula (I) according to Claim 1, in which R1 = R2 = R2' = R3 = H, X = C, n = m = 1 and R4 represents either a 4-imidazolyl group or a 5-methyl-4-imidazolyl group, for the preparation of a medicinal product for preventing or treating myocardial infarction, cardiac ischaemia, cardiac insufficiency, atherosclerosis, restenosis after PTCA or bypass, cerebral ischaemia and cerebral infarction, caused by an ischaemia, a trauma or a thromboembolic accident, Parkinson's disease, Huntington's chorea, acute renal insufficiency, graft rejection and accelerated atherosclerosis of grafts, inflammatory pathologies, immunological disorders, rheumatoid diseases, diabetes and pancreatitis, septic shock, acute respiratory distress syndrome, tumours and metastases, autoimmune diseases, AIDS, hepatitis, psoriasis, vasculitis, ulcerative colitis, multiple sclerosis and myasthenia.

11. Use of the compounds of formula (I) according to Claim 1, in which X represents a carbon atom, n, m, R1, R2, R2' and R3 are as defined in any one of Claims 1 to 4, and R4 represents a group -(CH₂)ₚ-heteroaryl, in which p may vary from 0 to 4 and in which the heteroaryl group is an imidazolyl group optionally substituted with a C1-C4 alkyl group, for the preparation of a medicinal product for preventing or treating myocardial infarction, cardiac ischaemia, cardiac insufficiency, atherosclerosis, restenosis after PTCA or bypass, cerebral ischaemia and cerebral infarction, caused by an ischaemia, a trauma or a thromboembolic accident, Parkinson's disease, Huntington's chorea, acute renal insufficiency, graft rejection and accelerated atherosclerosis of grafts, inflammatory pathologies, immunological disorders, rheumatoid diseases, diabetes and pancreatitis, septic shock, acute respiratory distress syndrome, tumours and metastases, autoimmune diseases, AIDS, hepatitis, psoriasis, vasculitis, ulcerative colitis, multiple sclerosis and myasthenia.

## Patentansprüche

1. Verbindungen der Formel (I) 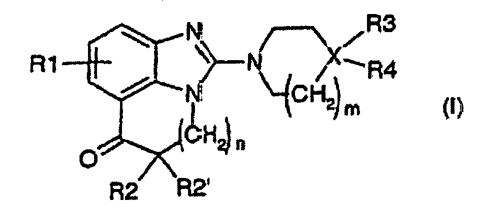 in der:
**R1** ein Wasserstoffatom, eine (C1-C4)-Alkylgruppe, ein Halogenatom, eine Nitrogruppe oder eine (C1-C4)-Alkoxygruppe bedeutet,
**R2** und **R2'** unabhängig voneinander ein Wasserstoffatom oder eine (C1-C4)-Alkylgruppe bedeuten,
**X** ein Stickstoffatom oder ein Kohlenstoffatom bedeutet,
**n** 1 oder 2 bedeutet,
**m** 1 oder 2 bedeutet,
**und dann, wenn X ein Stickstoffatom bedeutet:**
**R3** ein Wasserstoffatom oder eine (C1-C4)-Alkylgruppe bedeutet, zur Bildung der Verbindungen der Formel (I), die ein quaternäres Ammoniumatom aufweisen, oder nicht vorhanden ist zur Bildung der Verbindungen der Formel (I), die ein sekundäres oder tertiäres Amin umfassen,
**R4**:
ein Wasserstoffatom,
eine (C1-C6)-Alkylgruppe,
eine (C3-C7)-Cycloalkylgruppe,
eine (C3-C7)-Heterocycloalkylgruppe, die gegebenenfalls durch eine (C1-C4)-Alkylgruppe oder eine Gruppe -COOR substituiert ist, worin **R** eine (C1-C6)-Alkylgruppe darstellt,
eine -(CH₂)ₚ-Heteroarylgruppe, worin p einen Wert von 0 bis 4 aufweisen kann, worin die Heteroarylgruppe ausgewählt ist aus Pyridyl-, Aminopyridyl-, Pyrimidinyl-, Pyrazinyl-, Pyridazinyl-, Imidazolyl- und Thienylgruppen, wobei die Heteroarylgruppe gegebenenfalls durch eine (C1-C4)-Alkylgruppe substituiert ist,
eine Heteroarylcarbonylgruppe, wobei die Heteroarylgruppe ausgewählt ist aus Furyl-, Pyridyl-, Pyrimidyl-, Pyrazinyl-, Pyridazinyl- und Imidazolylgruppen,
eine Phenylcarbonylgruppe, wobei die Phenylgruppe gegebenenfalls durch ein Halogenatom substituiert ist,
eine (C1-C6)-Alkylcarbonylgruppe.
eine -(CH₂)ₚCOOR-Gruppe, worin p einen Wert von 0 bis 4 aufweisen kann und **R** eine (C1-C6)-Alkylgruppe darstellt,
eine Phenylsulfonylgruppe, die gegebenenfalls am Phenylkern durch ein Halogenatom, eine Trifluormethylgruppe, eine (C-C4)-Alkylgruppe, eine Nitrogruppe oder eine (C1-C4)-Alkoxygruppe substituiert ist, oder
eine -(CH₂)ₚ-Phenylgruppe, worin p einen Wert von 0 bis 4 aufweisen kann und worin die Phenylgruppe gegebenenfalls durch ein bis drei Gruppen substituiert ist, die unabhängig voneinander ausgewählt sind aus: einer (C1-C4)-Alkylgruppe, einer Nitrogruppe, einer Aminogruppe, einer Hydroxygruppe, einem Halogenatom, einer Trifluormethylgruppe, einer (C1-C4)-Alkoxygruppe, einer (C1-C4)-Alkoxyphenylgruppe, einer (C1-C4)-Alkylaminogruppe, einer Di-(C1-C4)-alkylaminogruppe, einer Gruppe -NHCHO oder -NHCOR', worin **R'** eine (C1-C4)-Alkoxygruppe oder eine (C1-C4)-Alkylgruppe darstellt, wobei die (C1-C4)-Alkylgruppe gegebenenfalls durch eine Dimethylaminogruppe substituiert sein kann, bedeutet,
**und in dem Fall, da X ein Kohlenstoffatom darstellt:**
**R3** ein Wasserstoffatom, eine Gruppe -NR5R6, eine Gruppe -(R(R5)₃⁺, eine Gruppe -NHCOR7, eine Gruppe -CONHR5, eine Gruppe COR7, eine Gruppe -NHCONH₂, eine Gruppe -OH oder eine Gruppe -CH₂OH darstellt,
**R4:**
ein Wasserstoffatom,
eine -(CH₂)ₚ-Phenylgruppe, worin p einen Wert von 0 bis 4 aufweisen kann und worin die Phenylgruppe gegebenenfalls durch ein bis drei Gruppen substituiert ist, die unabhängig voneinander ausgewählt sind aus einer (C1-C4)-Alkylgruppe, einer Nitrogruppe, einer Aminogruppe, einem Halogenatom, einer Trifluormethylgruppe oder einer (C1-C4)-Alkoxygruppe,
eine -(CH₂)ₚ-Heteroarylgruppe, worin p einen Wert von 0 bis 4 aufweisen kann und worin die Heteroarylgruppe ausgewählt ist aus einer Pyridylgruppe, einer Aminopyridylgruppe, einer Pyrimidinylgruppe, einer Pyrazinylgruppe oder einer Pyridazinylgruppe, oder
eine Gruppe -(CH₂)ₜNR7R8, worin t einen Wert von 0 oder 1 besitzt,
bedeutet,
mit der Maßgabe, daß, wenn R4 eine Gruppe -NR7R8 darstellt, R3 von den Gruppen -NR5R6, -NHCOR7, -NHCONH₂ und -OH verschieden ist,
**R5** und **R6** unabhängig voneinander ein Wasserstoffatom oder eine (C1-C4)-Alkylgruppe bedeuten.
**R7** und **R8** unabhängig voneinander eine (C1-C4)-Alkylgruppe oder (C1-C4)-Alkoxygruppe bedeuten oder gemeinsam einen gesättigten Ring bilden, der 5 bis 7 Kettenglieder umfaßt und gegebenenfalls ein zusätzliches Stickstoffatom enthält, wobei der Ring an einem Kohlenstoffatom oder einem Stickstoffatom, einschließlich dem Stickstoffatom, an das die Gruppen R7 und R8 gebunden sind, zur Bildung eines quaternären Ammoniumrests durch eine (C1-C4)-Alkylgruppe oder eine Gruppe -COOR", worin **R"** eine Phenylgruppe oder eine (C1-C4)-Alkylphenylgruppe darstellt, substituiert sein kann,
in Form der Enantiomeren, der Diastereoisomeren oder einer Mischung dieser verschiedenen Formen einschließlich der racemischen Mischungen sowie in Form der Basen oder der Additionssalze mit pharmazeutisch annehmbaren Säuren.

2. Verbindungen der Formel (I) nach Anspruch 1, **dadurch gekennzeichnet, daß**:
**R1** ein Wasserstoffatom, eine (C1-C4)-Alkylgruppe oder eine (C1-C4)-A1-koxygruppe darstellt,
**R2** ein Wasserstoffatom oder eine (C1-C4)-Alkylgruppe bedeutet,
**R2'** ein Wasserstoffatom bedeutet,
**X** ein Stickstoffatom bedeutet,
**n** 1 oder 2 bedeutet,
**m** 1 oder 2 bedeutet,
**R3** ein Wasserstoffatom darstellt, zur Bildung der Verbindungen der Formel (I), die eine quaternäre Ammoniumgruppe aufweisen, oder nicht vorhanden ist zur Bildung von Verbindungen der Formel (I), die ein sekundäres oder tertiäres Amin aufweisen,
**R4**:
ein Wasserstoffatom,
eine (C1-C6)-Alkylgruppe,
eine (C3-C7)-Cycloalkylgruppe,
eine Pyridyl-, Pyrimidinyl- oder Pyrazinylgruppe, die gegebenenfalls durch eine (C1-C4)-Alkylgruppe substituiert ist,
eine Heteroarylcarbonylgruppe, wobei die Heteroarylgruppe aus einer Furyloder einer Pyridylgruppe ausgewählt ist,
eine Phenylcarbonylgruppe, wobei die Phenylgruppe gegebenenfalls durch ein Halogenatom substituiert sein kann,
eine (C1-C6)-Alkylcarbonylgruppe,
eine Gruppe -(CH₂)ₚCOOR, worin p einen Wert von 0 bis 4 aufweisen kann und **R** eine (C1-C6)-Alkylgruppe darstellt.
eine Phenylsulfonylgruppe,
eine Phenylgruppe, die durch ein bis drei Gruppen substituiert ist, die unabhängig voneinander ausgewählt sind aus: einer (C1-C4)-Alkylgruppe, einer Nitrogruppe, einer Aminogruppe, einer Hydroxygruppe, einem Halogenatom, einer Trifluormethylgruppe, einer (C1-C4)-Alkoxygruppe, einer (C1-C4)-Alkoxy-phenylgruppe, einer Di-(C1-C4)-alkylaminogruppe, einer Gruppe -NHCHO oder -NHCO', worin **R'** eine (C1-C4)-Alkoxygruppe oder eine (C1-C4)-Alkylgruppe darstellt, wobei die (C1-C4)-Alkylgruppe durch eine Dimethylaminogruppe substituiert sein kann,
eine -(CH₂)ₚ-Phenylgruppe. worin p einen Wert von 0 bis 4 aufweisen kann,
eine -(CH₂)ₚ-Pyridylgruppe. worin p einen Wert von 0 bis 4 aufweisen kann,
eine -(CH₂)ₚ-Thienylgruppe. worin p einen Wert von 0 bis 4 aufweisen kann,
eine (C3-C7)-Heterocycloalkylgruppe, die gegebenenfalls durch eine (C1-C4)-Alkylgruppe oder eine Gruppe -COOR substituiert sein kann, worin **R** eine (C1-C6)-Alkylgruppe darstellt, bedeutet,
oder:
**R1** ein Wasserstoffatom bedeutet,
**R2** und **R2'** unabhängig voneinander ein Wasserstoffatom oder eine (C1-C4)-Alkylgruppe bedeuten,
**X** ein Kohlenstoffatom bedeutet,
**n** 1 oder 2 bedeutet,
**m** 1 bedeutet,
**R3** ein Wasserstoffatom, eine Gruppe -NR5R6, eine Gruppe -N(R5)₃⁺, eine Gruppe -NHCOR7, eine Gruppe -CONHR5, eine Gruppe -NHCONH₂, eine Gruppe -OH oder eine Gruppe -CH₂OH darstellt,
**R4:**
ein Wasserstoffatom,
eine Benzylgruppe,
eine Phenylgruppe, die gegebenenfalls durch ein bis drei Gruppen substituiert ist unabhängig ausgewählt aus: einer (C1-C4)-Alkylgruppe, einer Nitrogruppe, einer Aminogruppe, einem Halogenatom, einer Trifluormethylgruppe oder einer (C1-C4)-Alkoxygrugpe,
eine Pyridylgruppe,
eine Gruppe -NR7R8
bedeutet, mit der Maßgabe, daß, wenn R4 eine Gruppe -NR7R8 darstellt, R3 verschieden ist von den Gruppen -NR5R6, -NHCOR7, -NHCONH₂ und -OH,
**R5** und **R6** unabhängig voneinander ein Wasserstoffatom oder eine (C1-C4)-Alkylgruppe bedeuten,
**R7** und **R8** unabhängig voneinander eine (C1-C4)-Alkyl- oder (C1-C4)-Alkoxygruppe bedeuten oder gemeinsam einen gesättigten Ring bilden, der 5 bis 7 Kettenglieder aufweist und gegebenenfalls ein zusätzliches Stickstoffatom enthält, wobei der Ring an einem Kohlenstoffatom oder einem Stickstoffatom einschließlich dem Stickstoffatom, an das die Gruppen R7 und R8 gebunden sind, durch eine (C1-C4)-Alkylgruppe oder eine Gruppe -COOR", worin **R"** eine Phenylgruppe oder eine (C1-C4)-Alkylphenylgruppe bedeutet, substituiert sein kann zur Bildung einer quaternären Ammoniumgruppe,
in Form der Enantiomeren, der Diastereoisomeren oder von Mischungen dieser verschiedenen Formen einschließlich von racemischen Mischungen sowie in Form der Basen oder der Additionssalze mit pharmazeutisch annehmbaren Säuren.

3. Verbindungen der Formel (I) nach Anspruch 1 oder Anspruch 2, **dadurch gekennzeichnet, daß**:
**R1** ein Wasserstoffatom, eine Methylgruppe oder eine Methoxygruppe bedeutet,
**R2** ein Wasserstoffatom oder eine Methylgruppe bedeutet.
**R2'** ein Wasserstoffatom bedeutet,
**X** ein Stickstoffatom bedeutet.
**n** 1 oder 2 bedeutet,
**m** 1 oder 2 bedeutet,
**R3** ein Wasserstoffatom bedeutet zur Bildung der Verbindungen der Formel (I), die eine quaternäre Ammoniumgruppe aufweisen, oder nicht vorhanden ist zur Bildung der Verbindungen der Formel (I), die eine sekundäre oder tertiäre Aminogruppe aufweisen,
**R4**:
ein Wasserstoffatom,
eine (C1-C4)-Alkylgruppe,
eine (C6-C7)-Cycloalkylgruppe,
eine Pyridyl-, Pyrimidinyl- oder Pyrazinylgruppe, die gegebenenfalls durch eine (C1-C4)-Alkylgruppe substituiert ist,
eine Heteroarylcarbonylgruppe, wobei die Heteroarylgruppe aus einer Furyloder einer Pyridylgruppe ausgewählt ist,
eine Phenylcarbonylgruppe, wobei die Phenylgruppe gegebenenfalls durch ein Halogenatom substituiert sein kann,
eine (C3-C5)-Alkylcarbonylgruppe,
eine Gruppe -(CH₂)ₚCOOR, worin p einen Wert von 0 oder 1 besitzt und worin **R** eine (C1-C4)-Alkylgruppe darstellt,
eine Phenylsulfonylgruppe,
eine Phenylgruppe, die durch ein bis drei Gruppen substituiert ist, die unabhängig voneinander ausgewählt sind aus: einer Methylgruppe, einer Nitrogruppe, einer Aminogruppe, einer Hydroxygruppe, einem Halogenatom, einer Trifluormethylgruppe, einer Methoxygruppe, einer (C1-C4)-Alkoxy-phenylgruppe, einer Dimethylaminogruppe, einer Gruppe -NHCHO oder einer Gruppe -NHCOR', worin **R'** eine (C1-C4)-Alkoxygruppe oder eine (C1-C4)-Alkylgruppe darstellt, wobei diese (C1-C4)-Alkylgruppe durch eine Dimethylaminogruppe substituiert sein kann,
eine -(CH₂)ₚ-Phenylgruppe, worin p einen Wert von 1, 2, 3 oder 4 besitzt,
eine -(CH₂)ₚ-Pyridylgruppe, worin p einen Wert von 1 bis 3 besitzen kann,
eine -(CH₂)ₚ-Thienylgruppe, worin p den Wert 2 besitzt,
eine (C6-C7)-Heterocycloalkylgruppe, die gegebenenfalls durch eine Methylgruppe oder eine Gruppe -COOR substituiert ist, worin **R** eine (C1-C4)-Alkylgruppe darstellt, bedeutet,
oder:
**R1** ein Wasserstoffatom bedeutet,
**R2** und **R2'** unabhängig voneinander ein Wasserstoffatom oder eine Methylgruppe bedeuten,
**X** ein Kohlenstoffatom bedeutet,
**n** 1 oder 2 bedeutet,
**m** 1 bedeutet,
**R3** ein Wasserstoffatom, eine Gruppe -NR5R6, eine Gruppe -N(CH₃)₃^{*}, eine Gruppe -NHCOR7, eine Gruppe -CONHR5, eine Gruppe -NHCONH₂, eine Gruppe -OH oder eine Gruppe -CH₂OH bedeutet,
**R4**:
ein Wasserstoffatom,
eine Benzylgruppe,
eine Phenylgruppe, die gegebenenfalls durch ein bis drei Gruppen substituiert ist, die unabhängig voneinander aus einem Halogenatom oder einer Trifluormethylgruppe ausgewählt sind,
eine Pyridylgruppe,
eine Gruppe -NR7R8 bedeutet,
mit der Maßgabe, daß, wenn R4 eine Gruppe -NR7R8 bedeutet, R3 von den Gruppen -NR5R6, -NHCOR7, -NHCONH₂ und -OH verschieden ist,
**R5** und **R6** unabhängig voneinander ein Wasserstoffatom oder eine (C1-C4)-Alkylgruppe bedeuten, und
**R7** und **R8** unabhängig voneinander eine (C1-C4)-Alkylgruppe bedeuten oder gemeinsam einen gesättigten Ring bilden, der 5 bis 7 Kettenglieder aufweist und gegebenenfalls ein zusätzliches Stickstoffatom enthält, wobei der Ring an einem Kohlenstoffatom oder einem Stickstoffatom einschließlich dem Stickstoffatom, an das die Gruppen R7 und R8 gebunden sind, durch eine Methylgruppe oder eine Gruppe -COOR", worin **R"** eine (C1-C4)-Alkylphenylgruppe darstellt, substituiert sein kann zur Bildung einer quaternären Ammoniumgruppe,
in Form der Enantiomeren, der Diastereoisomeren oder von Mischungen dieser verschiedenen Formen einschließlich der racemischen Mischungen sowie in Form der Basen oder der Additionssalze mit pharmazeutisch annehmbaren Säuren.

4. Verbindungen nach einem der Ansprüche 1 bis 3, **dadurch gekennzeichnet, daß** sie ausgewählt sind aus:
. 1-(6-Oxo-5,6-dihydro-4H-imidazo[4,5,1-ij]chinolin-2-yl)-4-piperidinopiperidin
. 1-Methyl-1-[1-(6-oxo-5,6-dihydro-4H-imidazo[4,5,1-*ij*]chinolin-2-yl)-4-piperidinyl]-piperidin
. 2-[4-(4-Dimethylaminophenyl)-piperazin-1-yl]-4,5-dihydro-imidazo[4,5,1-*ij*]chinolin-6-on
. 2-[4-(4-*tert*.-Butoxycarbonylaminophenyl)-piperazin-1-yl]-4,5-dihydro-imidazo-[4,5,1-*ij*]chinolin-6-on
. 2-[4-(4-Aminophenyl)-piperazin-1-yl]-4,5-dihydro-imidazo[4,5,1-*ij*]chinolin-6-on
. 2-[4-(4-Acetamidophenyl)-piperazin-1-yl]-4,5-dihydro-imidazo[4,5,1-*ij*]chinolin-6-on
. 2-(4-n-Propyl-piperain-1-yl)-4,5-dihydro-imidazo[4,5,1-*ij*]chinolin-6-on
. 2-(4-*tert*.-Butyloxycarbonyl-piperazin-1-yl)-4,5-dihydro-imidazo(4,5,1-*ij*]chinolin-6-on
. 2-(Piperazin-1-yl)-4,5-dihydro-imidazo[4,5,1-*ij*]chinolin-6-on
. 2-(4-Acetyl-piperazin-1-yl)-4,5-dihydro-imidazo[4,5,1-*ij*]chinolin-6-on
. 2-[4-(4-Pyridyl)-piperazin-1-yl)-4, 5-dihydro-imidazo[4,5,1-*ij*]chinolin-6-on
. 2-(4-Benzyl-piperidin-1-yl)-4,5-dihydro-imidazo[4,5,1-*ij*]chinolin-6-on
. 1-(6-Oxo-5,6-dihydro-4*H*-imidazo[4,5,1-*ij*]chinolin-2-yl)-4-phenyl-4-piperidincarboxamid
. 1-(6-Oxo-5,6-dihydro-4*H*-imidazo[4,5,1-*ij*]chinolin-2yl)-4-phenyl-4-aminopiperidin
. 2-[4-(4-Hydroxyphenyl)-piperazin-1-yl]-4, 5-dihydro-imidazo[4,5,1-*ij*]chinolin-6-on
. 2-[4-(4-Fluorphenyl)-piperazin-1-yl]-4,5-dihydro-9-methyl-imidazo[4,5,1-*ij*]chinolin-6-on
. 1-[4-(4-Fluorphenyl)-piperazin-1-yl]-3-methyl-8,9-dihydro-7H-2,9a-diazabenzo-[cd]azulen-6-on
. 2-[4-(4-Fluorphenyl)-piperazin-1-yl]-4,5-dihydro-7-methyl-imidazo[4,5,1-*ij*]chinolin-6-on
. 2-[4-(4-Fluorphenyl)-piperazin-1-yl]-4,5-dihydro-8-methyl-imidazo[4,5,1-*ij*]chinolin-6-on
. 2-[4-(4-Fluorphenyl)-piperazin-1-yl]-4,5-dihydro-7-methoxy-imidazo[4,5,1-*ij*]-chinolin-6-on
. 2-[4-(4-Methoxyphenyl)-piperazin-1-yl]-4,5-dihydro-imidazo[4,5,1-*ij*]chinolin-6-on
. 1-(6-Oxo-5,6-dihydro-4*H*-imidazo[4,5,1-*ij*]chinolin-2-yl)-4H-4-dimethylaminopiperidin
in Form der Enantiomeren, der Diastereoisomeren oder von Mischungen dieser verschiedenen Formen einschließlich der racemischen Mischungen sowie in Form der Basen oder der Additionssalze mit pharmazeutisch annehmbaren Säuren.

5. Verfahren zur Herstellung einer Verbindung der Formel (I) nach einem der Ansprüche 1 bis 4, **dadurch gekennzeichnet, daß** man ein Derivat der Formel (II): 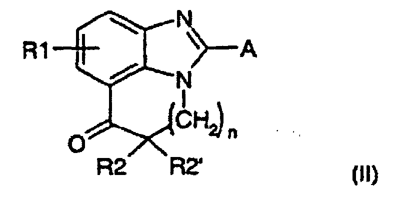 in der R1, R2, R2' und n die in Anspruch 1 angegebenen Bedeutungen besitzen und A eine austretende Gruppe darstellt, in Gegenwart eines Amins der Formel (III): 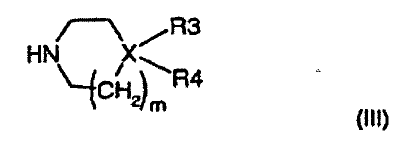 in der X, R3, R4 und m die in Anspruch 1 angegebenen Bedeutungen besitzen, in einem Lösungsmittel, welches ein Alkohol, ein Ether oder ein Kohlenwasserstoff sein kann, umsetzt zur Bildung einer Verbindung der Formel (I) nach Anspruch 1, wobei die Reaktion in Gegenwart einer Base, in Gegenwart von Alkalimetallhalogeniden oder in Gegenwart von Katalysatoren auf der Grundlage von Palladium oder Nickel durchgeführt werden kann.

6. Verbindungen der Formel (II), wie sie in Anspruch 5 definiert sind, nützlich als Synthesezwischenprodukte.

7. Arzneimittel, **dadurch gekennzeichnet, daß** es eine Verbindung der Formel (I) nach einem der Ansprüche 1 bis 4 enthält.

8. Pharmazeutische Zubereitung, **dadurch gekennzeichnet, daß** sie eine Verbindung der Formel (I) nach einem der Ansprüche 1 bis 4 sowie ein oder mehrere geeignete pharmazeutische Trägermaterialien enthält.

9. Verwendung einer Verbindung der Formel (I) nach einem der Ansprüche 1 bis 4 für die Herstellung eines Arzneimittels zur Vorbeugung oder Behandlung von Myokardinfarkt, Herzischämie, Herzinsuffizienz, Atherosklerose, Restenose nach PTCA oder Bypässen, Gehirnischämie und Gehirninfarkt, die durch Ischämie verursacht sind, Verletzungen oder thromboembolischen Vorfällen, neurodegenerativen Erkrankungen, wie die Parkinsonsche Krankheit, die Alzheimersche Krankheit, die Huntington Chorea, akuter Niereninsuffizienz, insbesondere ischämischen Ursprungs oder die nach der Nierentransplantation auftritt, bei der Herztransplantation, zur Behandlung der Abstoßung des Implantats und der beschleunigten Atherosklerose des Transplantats, Entzündungserkrankungen, immunologischen Störungen, rheumatoiden Erkrankungen, Diabetes und Pankreatitis, septischem Schock, akutem Atemnotsyndrom, Tumoren und Metastasen, Autoimmunkrankheiten, AIDS, Hepatitis, Psoriasis, Vaskulitis, Colitis ulcerosa, multipler Sklerose und Myasthenie.

10. Verwendung der Verbindungen der Formel (I) nach Anspruch 1, bei denen R1 = R2 = R2' = R3 = H, X = C, n = m = 1 und R4 entweder eine Imidazol-4-ylgruppe oder eine 5-Methyl-imidazol-4-yl-gruppe bedeutet, für die Herstellung eines Arzneimittels zur Vorbeugung oder Behandlung von Myokardinfarkt, Herzischämie, Herzinsuffizienz, Atherosklerose, Restenose nach PTCA oder Bypässen, Gehirnischämie und Gehirninfarkt, die durch Ischämie verursacht sind, Verletzungen oder thromboembolischen Vorfällen, der Parkinsonschen Krankheit, der Huntington Chorea, akuter Niereninsuffizienz, zur Behandlung der Abstoßung des Implantats und der beschleunigten Atherosklerose des Transplantats, Entzündungserkrankungen, immunologischen Störungen, rheumatoiden Erkrankungen, Diabetes und Pankreatitis, septischem Schock, akutem Atemnotsyndrom, Tumoren und Metastasen, Autoimmunkrankheiten, AIDS, Hepatitis, Psoriasis, Vaskulitis, Colitis ulcerosa, multipler Sklerose und Myasthenie.

11. Verwendung der Verbindungen der Formel (I) nach Anspruch 1, bei denen X ein Kohlenstoffatom bedeutet, n, m, R1, R2, R2' und R3 die nach einem der Ansprüche 1 bis 4 angegebenen Bedeutungen besitzen und R4 eine -(CH₂)ₚ-Heteroarylgruppe darstellt, worin p einen Wert von 0 bis 4 besitzen kann und worin die Heteroarylgruppe eine Imidazolylgruppe ist, die gegebenenfalls durch eine (C1-C4)-Alkylgruppe substituiert ist, für die Herstellung eines Arzneimittels zur Vorbeugung oder Behandlung von Myokardinfarkt, Herzischämie, Herzinsuffizienz, Atherosklerose, Restenose nach PTCA oder Bypässen, Gehirnischämie und Gehirninfarkt, die durch Ischämie verursacht sind, Verletzungen oder thromboembolischen Vorfällen, der Parkinsonschen Krankheit, der Huntington Chorea, akuter Niereninsuffizienz, zur Behandlung der Abstoßung des Implantats und der beschleunigten Atherosklerose des Transplantats, Entzündungserkrankungen, immunologischen Störungen, rheumatoiden Erkrankungen, Diabetes und Pankreatitis, septischem Schock, akutem Atemnotsyndrom, Tumoren und Metastasen, Autoimmunkrankheiten, AIDS, Hepatitis, Psoriasis, Vaskulitis, Colitis ulcerosa, multiple Sklerose und Myasthenie.
